# EUROPEAN PATENT SPECIFICATION

(11) **EP 3 806 939 B1**
(45) Date of publication and mention of the grant of the patent: **10.04.2024**
(21) Application number: 19819483.9
(22) Date of filing: 12.06.2019
(51) Int. Cl.: A61M 15/00

(54) **APPARATUS FOR USE IN DELIVERING RESPIRATORY DRUGS**
VORRICHTUNG ZUR VERWENDUNG BEI DER VERABREICHUNG VON ATEMWEGSMEDIKAMENTEN
APPAREIL POUR UTILISATION DANS L'ADMINISTRATION DE MÉDICAMENTS POUR LES VOIES RESPIRATOIRES

(30) Priority: 13.06.2018 AU 2018902108
(43) Date of publication of application: 21.04.2021
(73) Proprietor: Puff-Ah Pty Ltd, North Melbourne VIC 3051 (AU)
(72) Inventor: MISTRY, Jason, Yarraville, Victoria 3013 (AU); SIXSMITH, Edward, Jan Juc, Victoria 3228 (AU)
(74) Representative: Dehns
(86) International application number: PCT/AU2019/050603
(87) International publication number: WO 2019/237151

(56) References cited:
- WO-A1-92/00107
- WO-A1-95/20414
- WO-A1-96/37249
- WO-A1-2008/015542
- WO-A1-2009/153049
- WO-A1-2013/158738
- CA-C- 2 361 954
- DE-C1- 3 911 985
- US-A1- 2002 104 531
- US-A1- 2005 217 667
- US-A1- 2008 087 279
- US-B1- 6 416 743
- Robert McGlade: "Portable Asthma Spacer", coroflot , 20 July 2010 (2010-07-20), pages 1-13, XP055670710, Retrieved from the Internet: URL:https://www.coroflot.com/robmcglade/Po rtable-Asthma-Spacer [retrieved on 2019-05-13]

## Description

### Technical Field

The present invention relates to a collapsible inhaler for use with a metered dose inhaler (MDI) dispenser and further to systems of respiratory drug delivery.

### Background of the Invention

Respiratory drug delivery though the oronasal cavity is an established method of delivering medicaments containing pharmaceutically active agents into the pulmonary system. Many types of pharmaceutical agents can be delivered to act upon the lungs, and/or be absorbed into a patient's blood stream. Often the pharmaceutical agent is dispensed from a metered dose dispenser (MDI), commonly by action of a propellant. The canister is configured for hand operation and is calibrated to deliver a metered dose of pharmaceutical agent which is expelled as an aerosol, aiding in efficient adsorption of the pharmaceutically active agent once inside the patient's body.

In typical operation the pharmaceutical agent is expelled from the MDI canister in a concentrated manner directly into a patient's oronasal cavity, followed by inhalation to the pulmonary system. However, use of an intermediary stage referred to as a spacer, between patient and MDI allows for the expelled aerosol to expand and stabilise within the spacer prior to inhalation. Use of a spacer can lead to an improvement in the reliability of inhaled dose as it encourages extended and consistent inhalation. The increase in efficacious inhalation is also apparent in patients who have obstructed or inhibited lung function.

The intermediary stage can be an enclosed volume which allows for expansion and mixing of the pharmaceutical agent from the MDI dispenser with ambient air to deliver an aerosol with a more homogenised and more dilute concentration. The volume of medicament can then be inhaled by use of negative thoracic pressure from the patient. The volume required for the expansion of the propelled medicament in the spacer stage often requires the use of awkward and impractical devices which leads to misuse and difficulties in operation. Furthermore, the pharmaceutical agent can adhere to any surfaces leading to unreliability of dosage delivery and wastage of medicaments.

Conventional MDI dispensers in association with a spacer are often transported with patients for use when required, such as during sporting activities or other environments where inhalation of medicaments may be required. Such environments can include places that are likely trigger to allergic reactions including those close to animals, during periods of increased thunderstorm activity or of high pollen count. During transport, storage and in use patients can inadvertently actuate the MDI dispenser leading to wastage of medicament, unnecessary ware of their apparatus and residue build up which can require cleaning to restore full function.

In situations where delivery of medicament may be of some urgency, or through lack of ability for the unwell, or understanding in the case of paediatric or additional needs patients, a patient may not be able to take advantage of an spacer before attempting to actuate the MDI dispenser and inhale the medicament. Thus in situations such as these and similar a patient may not attempt to use an expanded spacer chamber and gain the benefit thereof.

It is therefore desirable to have a device which allows for inhalation of a pharmaceutically active agent dispensed from an MDI dispenser which ameliorates the aforementioned impracticalities required by expansion of the pressurised medicament and a system of respiratory drug delivery using the device. It is also desirable to have a device and system which encourage use of a spacer and prevent premature activation of an MDI dispenser.

A reference herein to other matters referred to as prior art is not to be taken as an admission that the document or matter was known or that the information it contains was part of the common general knowledge as at the priority date of any of the claims. DE 39 11 985 describes an inhaler with a spray tube leading to a mouthpiece which can be pivoted relative to the cartridge housing from an axially aligned position to a position of use angled in an L-shape.
WO 96/37249 describes a respiratory apparatus for use in an inhaler system, including a collapsible chamber with a mouthpiece.

### Summary of Invention

The invention is defined by the appended claims. According to one aspect of this invention there is provided a collapsible inhaler for use with a metered dose inhaler (MDI) dispenser that is operable to dispense a metered dose of medicament therefrom, the inhaler comprising, an inlet member being adapted to receive an MDI dispenser and having an inlet therein; an outlet member comprising an outlet therein, a conduit which is pliable and has a distal end associated with the inlet member and a proximal end associated with the outlet member, a support associated with the pliable conduit that is adjustable between an expanded state and a contracted state whereby in the contracted state the inlet member is positioned proximate to the outlet member, while in the expanded state the support supports the pliable conduit and combined with the inlet member being spaced from the outlet member that defines a chamber, so that in use a metered dose of medicament can be dispensed from the MDI dispenser through the inlet in the inlet member to mix with air in the chamber and be inhaled by a patient through the outlet in the outlet member.

It will be appreciated that in the contracted state the inhaler occupies less volume providing a state which is practical for ease of transport, storage and manipulation. The expanded state provides, conveniently on demand, a chamber as defined for mixing of the medicament with ambient air within to provide advantageous delivery of inhalation of a metered dose of pharmaceutically active agent contained within medicament dispensed from the MDI dispenser. These advantages include but are not limited to, reliability of dosage delivered to the patient, consistent, extended and deeper inhalation and overall reliability of respiratory drug delivery efficacy. In the expanded state, the chamber further slows the ejected medicament resulting in decreased deposition of medication in the oropharynx whose effect is both more reliable pulmonary delivery as well as decreased risk of side effects, such as hoarse voice. The use of an inhaler with a chamber also negates the requirement for coordination of MDI actuation with respiration, especially useful in the young, additional needs or unwell patients.

The support conveniently supports the pliable conduit and the outlet member in the expanded state allowing the patient to hold and actuate the device using the inlet member, the outlet member is further configured to allow inhalation through a patient's mouth into the pulmonary system. The pliability of the supported conduit enables collapsing of the device to decrease in volume providing the contracted state. In some embodiments the support is such that in the expanded state the pliable conduit is held taut and such that the weight of the outlet member is supported without substantial deformation of the chamber. The conduit, outlet member and inlet member define a chamber, having a cavity volume wherein the medicament mixes. The pliable conduit can be formed of any deformable material, preferably a durable plastic.

In a further set of embodiments, the inhaler further comprises a lockout mechanism for inhibiting operation of the MDI dispenser when the support and conduit are in the contracted state. The mechanism does not inhibit operation of the MDI when the support and conduit are the expanded state.

According to another aspect of the invention there is provided a collapsible inhaler for use with a metered dose inhaler (MDI) dispenser that is operable to dispense a metered dose of medicament therefrom, the inhaler comprising: an inlet member being adapted to receive an MDI dispenser and having an inlet therein; an outlet member comprising an outlet therein, a conduit which is pliable and has a distal end associated with the inlet member and a proximal end associated with the outlet member, a support associated with the pliable conduit that is adjustable between an expanded state and a contracted state whereby in the contracted state the inlet member is positioned proximate the outlet member, while in the expanded state the support supports the pliable conduit and combined with the inlet member being spaced from the outlet member that defines a chamber, so that in use a metered dose of medicament can be dispensed from the MDI dispenser canister through the inlet in the inlet member to mix with air in the chamber and be inhaled by a patient through the outlet in the outlet member, wherein the inhaler further comprises a lockout mechanism for inhibiting operation of the MDI dispenser when the support and conduit are in the contracted state and not inhibiting operation of the MDI dispenser when the support and conduit are in the expanded state.

In further embodiment the MDI dispenser comprises an MDI canister containing the medicament and a needle valve for dispensing the medicament there through, wherein the valve opens upon depression of the MDI canister by engagement with a saddle associated with the inlet member.

In yet further embodiments the MDI dispenser comprises an MDI canister containing the medicament and a needle valve for dispensing the medicament there through, and further comprises an actuator comprising a saddle and a mouth piece, wherein the valve of the MDI canister opens upon depression of the MDI canister by engagement with the actuator saddle. The lockout mechanism prevents depression of the MDI dispenser when the support and conduit is in the contracted state by engaging with the MDI dispenser and preventing actuation. Thus the lockout mechanism prevents depression of the MDI dispenser when the support and conduit is in the contracted state by engaging with the MDI dispenser and preventing actuation. The lockout mechanism acts as wedge acting upon the MDI canister and saddle of the inlet member. Alternatively, the lockout mechanism or MDI canister and actuator saddle, to prevent actuation thereof when the support and conduit are in the contracted state. Conversely, the lockout mechanism and does not inhibit depression of the MDI canister when the support and conduit are in the expanded state.

In one set of embodiments the inhaler is adapted for use with an MDI dispenser comprising an MDI canister where the lockout mechanism is comprised of an elongate member extending from an interior surface of the outlet member towards the inlet member to engage and act as a wedge between the MDI canister and the saddle of the inlet member inhibiting depression of the MDI canister and operation when the support and conduit are in the contacted state. Conversely the elongate member does not engage and act as a wedge between the MDI canister and inlet member saddle such that it does not inhibit depression of the MDI canister when the support and conduit are in the expanded state. The skilled addressee will recognise that many alternative arrangements can be used to perform this function.

In a further set of embodiments the inhaler is adapted for use with an MDI dispenser comprising an MDI canister and an actuator wherein the actuator comprising a saddle and a mouth piece, wherein the valve of the MDI canister opens upon depression of the MDI canister by engagement with the actuator saddle, wherein the lockout mechanism inhibits depression of the MDI when the support and conduit are in the contracted state and does not inhibit depression of the MDI when the support and conduit are in the expanded state. The lockout mechanisms as described provide several advantages, such as inhibiting actuation of the MDI dispenser whilst the device is in the contracted state, therefore coercing the patient to use the device in the expanded state and obtain the advantages provided by the expanded state as discussed. Thus this mechanism, in combination with the convenience of a collapsible spacer, provides a safe and effective way of ensuring a patient, such as a child, unwell or one of additional needs, make use of the spacer whilst also providing quick access to their medicament. A inhaler such as this is well suited to use in sporting, activities and environments where thunderstorm asthma events, or high pollen counts are common. Furthermore, whilst in the collapsed state, the lockout mechanism prevents unintended actuation of the MDI dispenser during storage, transport and manipulation reducing wastage of medicament and wear to the MDI dispenser.

In another set of embodiments, the inhaler includes a mouth piece associated with the outlet member, in some embodiments the mouthpiece is extendible. The mouth piece provides a convenient means to provide an at least partial seal with the patient's mouth to enable inhalation of the medicament by use of negative thoracic pressure. Other suitable means to achieve this end include but are not limited to pipes, apertures, straws and the like. Having an extendable mouth piece further enhances the collapsibility of the device, enhancing the practical advantages of the device as discussed. In further embodiments the mouth piece is biased to be extended from an exterior surface of the outlet member and the mouthpiece is retained by a cover, enabling the mouth piece to pop-out on removing of the cover.

In some embodiments the mouth piece cover is mounted by a hinge on the exterior surface of the inlet member and the cover releasably engages with the exterior surface of the outlet member, release of the cover allows for extension of the mouth piece. The mouth piece may be mounted in by any means capable, including for example, being mounted in parallel tracks to allow the mouth piece to slide there between, or removable from the device and being mounted by frictional engagement. The mouth piece cover protects the mouth piece from the environment and improves hygienic aspects of the device.

In a further embodiment of the invention the inlet member and outlet member are adapted to realisably engage each other when the support and conduit are in the contracted state. The releasable engagement provides a convenient way of maintaining the contracted state. The skilled person will recognise that many types of latches, locks and other frictionally engaging arrangements can perform this function. In a further embodiment the support is biased towards the extended state such that expansion of the collapsible device occurs on release of the engaging arrangement.

In some embodiments the, an arrangement for achieving this end is by way of a bayonet fitting. The inlet member further comprises at least one lug; and the outlet member further comprises a lip having at least one recess aperture to receive the lug, the recess aperture radially traversing around the circumference of lip such that engagement of the lug is rotational in manner. It will be appreciated that this arrangement may be reversed; such that the lug(s) are positioned on the out let member and the recess(es) on the inlet member and vice versa. In some embodiments the a lip, flange, skirt or any other load baring projection may be used to house the engagement means. Furthermore the lug may be comprised of any loop, bump, raised area capable of engaging with the recess in a manner that is releasable. In some embodiments the device has three lugs and apertures which are situated at 120° points around the circumference of the inlet member and outlet member.

In further embodiments, the outlet member further comprises a grip incorporated into or mounted on the outlet member. In some embodiments the grip is formed of an over-moulded rubber or silicone. The grip aids in the user gripping the outlet member, especially in a particular embodiment a twisting motion is required to disengage the bayonet locking engagement which may be especially useful for young or unwell patients.

In a further set of embodiments of the invention the inlet member is adapted to receive an MDI canister. An adaptation suitable to receive the MDI canister is by way of a tube shaped appendage that is complementary in shape to the MDI canister such that an at least partial seal is formed in use. The tube being of such a complimentary size and such that the MDI canister fits in with enough clearance to be removable, but tight enough to provide an at least partial seal. In further embodiments the inlet member further comprises a lip for associating with the distal end of conduit and support. In this set of embodiments the saddle for actuating the MDI canister by engagement with the needle valve is associated with the inlet member. A skilled person will recognise that any shape of appendage that is at least partially complementary to the shape of an MDI canister can be suitable to receive the MDI canister.

In further embodiments the inlet member further comprises a first association portion for association with the distal end of the support and conduit. Association of the distal end of the conduit and support to the first association portion may be achieved by any suitable means including use of frictionally engaging o-ring, adhesive, screws or by forming the conduit integrally from the inlet member. Most preferably association is achieved using a frictionally engaging o-ring.

In a particular embodiment, the tube shaped appendage can be formed of two halves which enclose a mount. Most preferably the mount is cylindrical in shape, although any shape capable of forming an at least partial seal at the distal end of the conduit may be used, such as continuous surfaces including ovoid and the like. The halves of the tube shaped appendage can join to give a circular portion to enclose the mount and a tube shaped appendage to receive the MDI dispenser. Preferably the tube shaped appendage provides an at least partial seal with the body of the MDI dispenser, the seal may be provided by addition of a resiliently deformable annular seal. An alternate embodiment is that the appendage is integrally formed; another alternate is that the appendage and mount are all integrally formed.

In some embodiments the cylindrical mount comprises a load bearing section for alternatively attaching the distal end of the conduit and support which may take the form of a lip, flange, skirt or any other suitable shape. In these embodiments, association of the distal end of the conduit and support to the load bearing section of the mount may be achieved by any suitable means including use of frictionally engaging o-ring, adhesive, screws or by forming the conduit integrally from the inlet member. Most preferably association is achieved using a frictionally engaging o-ring, this attachment method is particularly suitable for embodiments having substantially ovoid cross section.

In some embodiments the mount further comprises a saddle to engage a needle valve of the MDI canister, at an angle relative to the conduit. Preferably the angle of the MDI canister relative to the conduit leads to release of the medicament substantially parallel to the conduit. Releasing of the medicament aerosol upon actuation of the MDI canister in a substantially parallel manner to the chamber directs the ejected aerosol into the chamber prior to inhalation, the direction of flow being towards the chamber and substantially parallel to an axis of the chamber having the advantage of not being directed towards any of the immediate surfaces forming the perimeter of the chamber, thus reducing adherence of the medicament. The mount can further comprises a series of radially extending spokes from a centre of locos of the mount to a circular rim. The saddle for engaging the MDI canister needle value being positioned at the centre of locus where the spokes meet. Any number and shape of spoke can be used as will be understood by a skilled person.

In a further set of embodiments the inlet member is adapted to receive an MDI dispenser comprising an MDI canister, an actuator comprising a saddle for actuating the MDI canister and mouthpiece. In this set of embodiments the interior of the inlet member is shaped such that the MDI is received within a tube shaped appendage. The MDI can be held in place by frictional engagement, the inner surface of the tube may be provided with rubber or other suitably resilient material to aid in said engagement. Alternatively a frictionally engaging o-ring can be used. A skilled person will recognise that any shape of appendage that is at least partially complementary to the shape of an MDI can be suitable to receive the MDI. In such a set of embodiments, the inhaler can be at least partially disassembled before insertion of the MDI prior to use. It is at this point it is convenient to state that an addition advantage of several embodiments of the invention allow for ready assembly and disassembly of the inhaler. Such ready assembly and disassembly enables convenient cleaning of the inhaler.

In yet further embodiments the inlet member is adapted to receive an MDI dispenser comprising an MDI canister, an actuator comprising a saddle for actuating the MDI canister and mouthpiece. In some embodiments the adaptation is by way of having a complementary shaped recess the recess comprising an aperture traversing from an exterior surface of the inlet member into the interior of the inlet member and in communication with the chamber. The recess also having a shape complementary a mouthpiece that of an MDI. Thus the mouthpiece of the MDI is received in the recess in fractional engagement where the MDI is readily removable whilst also capable of forming an at least partial seal when in use. In further embodiments the recess is shaped such that the MDI releases the medicament relative to the conduit substantially parallel to an axis of to the conduit.

In further embodiments, the outlet member is lenticular in shape having at a concave surface facing interior to the chamber. Most preferably the shape is lenticular as highly continuous and smooth surfaces have less area for medicament to become adhered to. The concave surface having a second association portion for associating with the proximal end of the conduit and support. Association of the proximal end of the conduit and support may be achieved by any suitable means including use of frictionally engaging o-ring, adhesive, screws or by forming the conduit integrally from the inlet member. Most preferably association is achieved using a frictionally engaging o-ring.

In further embodiments, the device comprises at least one vent which traverses the inlet of the inlet member through to the distal end of the chamber. The at least one vent may is preferably positioned such that the vent allows for mixing of air and enabled by air flow during inhalation as negative thoracic pressure causes the chamber of the device to also experience negative pressure. The vent allows for exterior air to enter the chamber, thus allowing air to flow into the chamber and carrying and mixing the medicament into the respiratory system of the patient. It will be appreciated that the at least one vent may be positioned anywhere in communication with both the interior chamber and inlet of the inlet member. Most preferably the least one vent is a one way valve, opening into the chamber from the exterior. The advantage of the one way value is to prevent substantial leakage of medicament, after actuation of the MDI dispenser and before inhalation from the patient, and further aiding in mixing of the medicament aerosol during inhalation.

In further embodiments, the mouth piece further comprises at least one outflow vent directing any expired air into the surrounding atmosphere. The outflow vent further prevents expired mixed air from entering the chamber. It will be appreciated that the outflow vent can be situated at any suitable location on the mouth piece, and any suitable number of vents can be used. Preferably the outflow vent traverses from a cavity in formed in the mouth piece, where the mouth is received, to an exterior of the device. Most preferably the device comprises four outflow vents radially dispersed around the mouth piece at 90° intervals.

In some embodiments, the mouth piece is traversed by a one way duck bill valve. Preferably, the one way valve opening from chamber to exterior on application of negative thoracic pressure. The valve preventing unintended escape of medicament after MDI is actuated and before inhalation.

In some embodiments the support is a helical spring, although any suitable spring configuration may be used. Any device capable of storing kinetic energy to expand may be used as a suitable support such as a folded arrangement or concertinaed arm. A spring has the advantage of compressing into a confined space, thus minimising the volume of the contracted state.

Most preferably the spring has a tension such that expansion of the conduit and outlet member between the collapsed and expanded state by the spring is about linear along an axis parallel to the chamber. Thus giving rise to a chamber that is substantially linear along an axis parallel to the chamber in the expanded state, such that the weight of the outlet member and inlet member do not substantially deform the conduit in the expanded state. The preferred tension enables the pliable conduit to be taut in the extended state and provides the necessary tension to support the outlet member without substantial deformation of the chamber. Further, the tension of the spring is not so high that collapsing of the inhaler into the collapsed state is not overly burdensome for the operator, specially paediatric patents and those of additional needs. The spring tension also is such that it permits safe expansion from the collapsed to extended state.

In some embodiments the conduit and support are adapted to prevent adherence of the pharmaceutically active agent. Such adaptations include, the interior of the conduit being smooth, featureless or continuous inner surface forming part of the chamber which prevents physical adherence of the pharmaceutically active agent in discontinuities in the pliable conduit. The surface may also have a polish, gloss or be coated by an anti-static or non-stick material preventing adherence of the pharmaceutically active agent. Most preferably all surfaces exposed to the medicament containing the pharmaceutically active agent are adapted as discussed to prevent adherence, including the support, outlet member, inlet member, duck billed valve and the mouth piece.

Such anti-statics include anti-static coating such as silicone, Sikafloor^{®} n long-chain aliphatic amines (optionally ethoxylated) and amides, quaternary ammonium salts (e.g., behentrimonium chloride or cocamidopropyl betaine), esters of phosphoric acid, polyethylene glycol esters, or polyols. Examples of non-stick coatings include Teflon, Polytetrafluoroethylene (PTFE), Silverstone (plastic), a three- layer coating using PTFE and PFA, Superhydrophobic coatings, Liquid-impregnated surface such as LiquiGlide. Most preferably the surfaces contacting the medicament have a surface resistivity of about 1×10¹² Ohms/square or less as measured according to ASTM D257-07.

In some embodiments the conduit is cylindrical in shape, although the conduit may take any form suitable for use. Preferably the conduit is tapered. Preferably the taper is between 0.5 to 20%, 1% to 10%, more preferably the taper is between 2% and 7%, most preferably the taper is up to and including 5% of the circumference, most preferably the circumfluence is between 10 mm and 500 mm, 40 mm to 250 mm at the inlet member and/or outlet member. Most preferably the circumfluence about 52 mm at the inlet member and about 50 mm at the outlet member.

In some embodiments the volume of the chamber is about from about 8000 mL to 10 mL, preferably 6000 mL to 20 mL, more preferably 2000 mL to 40 mL, even more preferably 100 to 500 mL, most preferably 150 mL to 400 mL, and the most preferable volume is about 200 mL.

In further embodiments the conduit comprises an anti-static material. Any pliable material that is capable of collapsing may be used to form the conduit. Examples of suitable material include plastics polyethylene, polyester, polyurethane, nylon, silicone coated polyethylene, Cordura. Most preferably the material is nylon based including Cordura.

In some embodiments the inhaler is adapted for paediatric use, such adaptations include a chamber volume of suitable size to match a child's lungs; a mouth piece of suitable size for their mount; and a spring of a tension capable of being collapsible by a child' strength.

According to another aspect of the invention there is provided a system for respiratory delivery of a medicament, the system comprising an inhaler as hereinbefore described and an MDI dispenser, wherein the MDI dispenser contains the medicament, wherein the medicament composition comprises the pharmaceutically active agent.

In some embodiments of the system operation of the MDI dispense when the support and conduit are in the contracted state and operation of the MDI dispenser not inhibited when the support is in the expanded state. Advantageously the lockout mechanism prevents use of the inhaler when in the contracted state. When in the expanded state the patient receives the advantages of conveniently having the chamber as defined for mixing of the medicament with ambient air within to provide advantageous delivery of any pharmaceutically active agents contained within the medicament. These advantages include but are not limited to, more relatable dosage delivery, inhalation and overall reliability of respiratory drug delivery efficacy. In the contracted state the system occupies less volume providing a state which allows practical for ease of transport, storage and manipulation.

In some embodiment the composition further comprises a propellant, a propellant is to be understood as compressible substance which is not substantially toxic and preferably does not cause undue environmental harm. In preferred embodiments the propellant is a fluoroalkane. Common fluoroalkanes include 1,1,1,2-tetrafluoroethane. In the compositions to be delivered with an MDI dispenser the propellant is preferably a hydrofluorocarbon selected from the group of HFA 134a, HFA CF₃CHFCF₃, also known as HFA 227, HFC 227 or 1,1,1,2,3,3,3- heptafluoropropane.

The system is suitable to deliver any pharmaceutically active agent which can be delivered into the respiratory system. Examples of pharmaceutically active agents delivered into the respiratory system include, anticholinergics, beta-adrenergic agonists, corticosteroids, leukotriene inhibitors, mast cell stabilizers, phosphodiesterase 4 (PDE4) inhibitors, inhaled magnesium sulphate and mixtures thereof.

Preferably, the system delivers pharmaceutically active agents selected from the list comprising: short-acting beta-adrenergic agonists, corticosteroids, tocolytics, long-acting beta-adrenergic agonist or mixtures thereof. Most preferably the system is used for delivering respiratory drugs for the prophylaxis and/or treatment of asthma and related disorders characterized by symptoms such as reversible airflow obstruction, and bronchospasm. Such disorders may further include chronic obstructive pulmonary disease (COPD) and transient bronchospasm relating from allergens as might be experienced during thunderstorm asthma events.

Even more preferably the system is adapted for paediatric use. Such adaptations include the mouth piece and volume of the chamber being of a suitable size to suit younger patient's lung capacity, mouth size and strength for collapsing the support and conduit to give the collapsed state. Furthermore the adjustable support and conduit is user friendly and enables children of most ages to expand and collapse independently. In further embodiments, the outlet member further comprises a grip incorporated into or mounted onto the outlet member. Preferably the grip is formed of an over-moulded rubber or silicone. The grip aids in the user gripping the outlet member. Especially in a preferred embodiment where a twisting motion is required to disengage the bayonet locking engagement which may be especially useful for young and/or unwell patients.

According to another aspect, which is not claimed, there is provided a method of respiratory administration of a medicament to a subject in need thereof, wherein the medicament composition comprises a pharmaceutically active agent, comprising providing the inhaler as hereinbefore described, inserting an MDI dispenser into the inlet member; operating the MDI dispenser to dispense a metred dose of the medicament through the inlet of the inlet member into the chamber, wherein the medicament is dispersed within the volume of the chamber; and inhaling the medicament through the outlet out the outlet member.

In some embodiments, the operation of the MDI dispenser is inhibited when the support is in the contracted state and operation of the MDI dispenser not inhibited when the support is in the expanded state. Advantageously the lockout mechanism prevents use of the inhaler when in the contracted state. When in the expanded state the patient receives the advantages of conveniently having the chamber as defined for mixing of the medicament with ambient air within to provide advantageous delivery of any pharmaceutically active agents contained within the medicament. These advantages include but are not limited to, more relatable dosage delivery, inhalation and overall reliability of respiratory drug delivery efficacy.

In some embodiment the composition further comprises a propellant, a propellant is to be understood as compressible substance which is not substantially toxic and preferably does not cause undue environmental harm. In preferred embodiments the propellant is a fluoroalkane. Common fluoroalkanes include 1,1,1,2-tetrafluoroethane. In the compositions to be delivered with an MDI dispenser the propellant is preferably a hydrofluorocarbon selected from the group of HFA 134a, HFA CF₃CHFCF₃, also known as HFA 227, HFC 227 or 1,1,1,2,3,3,3- heptafluoropropane.

In further examples the method delivers any pharmaceutically active agent which is suitable to be delivered into the respiratory system examples of drugs delivered by the respiratory system include, anticholinergics, beta-adrenergic agonists, corticosteroids, leukotriene inhibitors, mast cell stabilizers, phosphodiesterase 4 (PDE4) inhibitors, inhaled magnesium sulphate and mixtures thereof.

Preferably, the method delivers pharmaceutically active agents selected from the list comprising: short-acting beta-adrenergic agonists, corticosteroids, tocolytics, long-acting beta-adrenergic agonist or mixtures thereof. Most preferably the system is used for delivering respiratory drugs for the prophylaxis and/or treatment of asthma and related disorders characterized by symptoms such as reversible airflow obstruction, and bronchospasm. Such disorders may further include chronic obstructive pulmonary disease (COPD).

More preferably the method delivers a short-acting beta-adrenergic agonist selected from the list comprising salbutamol, pirbuterol, Ipratropium, budesonide, terbutaline, levosalbutamol, salmeterol, formoterol, pharmaceutically active salt, pharmaceutically derivative or combinations thereof. Most preferably the delivered pharmaceutically active agent is a short-acting beta-adrenergic agonist being salbutamol.

Even more preferably the system is adapted for paediatric use. Such adaptations include the mouth piece and volume of the chamber being of a suitable size to suit younger patient's lung capacity, mouth size and strength for collapsing the support and conduit to give the collapsed state. Furthermore the adjustable support and conduit is user friendly mechanism enables children of most ages to expand and collapse independently.

In yet a further embodiment the device further comprises a counter for monitoring doses of medication administered. The counter functions as a roller counter with an increase of one per each activation of the inhaler and may be reset. The counter allows the user to count how much of each medication canister has been used. Preferably the counter is mounted to the inlet member.

As used herein and as is understood in the art an MDI dispenser can comprise an MDI canister containing medicament. The MDI canister comprises a needle valve, wherein the valve of the MDI canister opens upon depression of the MDI canister by engagement with a saddle such that valve opens to dispense a metered dose of medicament. An MDI dispenser can further comprise an actuator comprising a saddle and a mouth piece with the actuator saddle. The MDI canister contains the medicament which comprises a composition comprising the pharmaceutically active agent and often a pressurized propellant.

Where the terms "comprise", "comprises" and "comprising" are used in the specification (including the claims) they are to be interpreted as specifying the stated features, integers, steps or components, but not precluding the presence of one or more other features, integers, steps or components, or group thereof.

As used herein, the terms "first", "second", "third" etc in relation to various features of the disclosed devices are arbitrarily assigned and are merely intended to differentiate between two or more such features that the device may incorporate in various embodiments. The terms do not of themselves indicate any particular orientation or sequence. Moreover, it is to be understood that the presence of a "first" feature does not imply that a "second" feature is present, the presence of a "second" feature does not imply that a "first" feature is present, etc.

It will be convenient to hereinafter describe the invention in greater detail by reference to the accompanying drawings. The detailed description and the drawings are however merely illustrative of how the invention might be put into effect, so that the specific form and arrangement of the various features as shown is not to be understood as limiting on the invention.

### Brief Description of Drawings

Figure 1 is an isometric view of a preferred embodiment when the conduit and support of the device are in the collapsed state according to one aspect of the invention.
Figure 2 is a side view of a preferred embodiment when the conduit and support of the device are in the collapsed state according to one aspect of the invention.
Figure 3 is a top plan view of a preferred embodiment when the conduit and support of the device are in the collapsed state according to one aspect of the invention.
Figure 4 is a sectional side view through plane I of figure 1 of a preferred embodiment when the conduit and support of the device are in the collapsed state according to one aspect of the invention.
Figure 5 is a sectional inverse plan view through II of figure 3 of a preferred embodiment when the conduit and support of the device are in the collapsed state according to one aspect of the invention.
Figure 6 is an isometric view of a preferred embodiment when the conduit and support of the device are in the collapsed state according to one aspect of the invention with mouthpiece released.
Figure 7 is a sectional side view through plane III of figure 6 of a preferred embodiment when the conduit and support of the device are in the collapsed state according to one aspect of the invention with mouthpiece released.
Figure 8 is an expanded view of detail "J", a preferred embodiment of lock out the mechanism when the conduit and support of the device are in the collapsed state according to one aspect of the invention.
Figure 9 is a sectional inverse plan view through plane IV of figure 6 of a preferred embodiment when the conduit and support of the device are in the collapsed state according to one aspect of the invention with mouthpiece released.
Figure 10 is an isometric view of a preferred embodiment of when the conduit and support of the device are in the expanded state according to one aspect of the invention with mouthpiece released.
Figure 11 is a side view of a preferred embodiment of when the conduit and support of the device are in the expanded state according to one aspect of the invention with mouthpiece released.
Figure 12 is a plan view of a preferred embodiment of when the conduit and support of the device are in the expanded state according to one aspect of the invention with mouthpiece released. The along an axis parallel to the chamber is shown in this drawing by arrow 196.
Figure 13 is a sectional view through plane V of figure 11 of a preferred embodiment of when the conduit and support of the device are in the expanded state according to one aspect of the invention with mouthpiece released.
Figure 14 is an expanded view of detail "E", a preferred embodiment of lock out the mechanism of when the conduit and support of the device are in the expanded state according to one aspect of the invention.
Figure 15 is an isometric view of a preferred embodiment of when the conduit and support of the device are in the expanded state according to one aspect of the invention with mouthpiece released with MDI dispenser depressed.
Figure 16 is a sectional side view through plane VI of figure 15 of a preferred embodiment of when the conduit and support of the device are in the expanded state according to one aspect of the invention with mouthpiece released with MDI depressed.
Figure 17 is an expanded view of detail "F", a preferred embodiment of lockout the mechanism of when the conduit and support of the device are in the expanded state with the MDI depressed according to one aspect of the invention.
Figure 18 is an isometric, exploded view of a preferred embodiment of the device according to one aspect of the invention.
Figure 19 is an isometric view and a plan view of a preferred embodiment of when the conduit and support of the device are in the collapsed state, with the mouth piece open, showing bayonet fittings according to one aspect of the invention.
Figure 20 is a perspective view of a preferred embodiment when the conduit and support are in the contracted state, with the mouth piece extended, showing the preferred arrangement of the outflow vents.

### Detailed Description

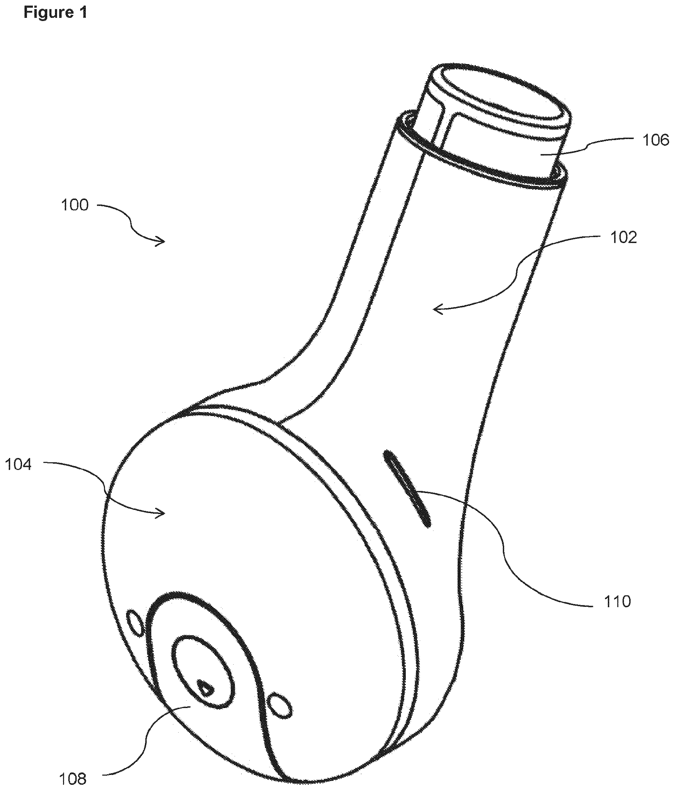

Figure 1 illustrates a preferred embodiment of the device when the support and conduit are in the collapsed state 100 according to one aspect of the invention. The device 100 comprises an inlet member 102, which is adapted to receive and actuate an MDI dispenser, such as MDI canister 106 or MDI canister and actuator 196, the dispenser is shown in a non-actuated position, an MDI dispenser can be actuated by depression or downward pressure from the operator. The dispenser, such as canister 106 is interchangeable and upon actuation dispenses a metered dose of medicament. The inlet member 102 may take any form suitable to receive the dispenser and allow for transfer of the medicament from the dispenser 106 into the interior of the device. The tube shaped appendage 182 being of such a complimentary size and such that the MDI canister 106 or MDI canister and actuator 196 fits in with enough clearance to be removable, but tight enough to provide an at least partial seal

Further shown is the outlet member 104, the outlet member being configured to have an outlet for inhaling the medicament. The outlet member 104 as shown is preferably lenticular in shape, but any form suitable can be used. The device contains a vent 110, the vent traverses from exterior the device into the interior of the device and is preferably a one way vent operating in a direction from an exterior to interior of the device. The vent operates to allow air into the device and to flow through upon use through negative thoracic pressure through the mouth piece 122. The vent 110 enables equalisation of the concurrent negative pressure in the chamber thus enabling inhalation whilst not allowing substantial leakage of the medicament. The vent 110 is shown as a preferred embodiment as part of the inlet member, although any number of vents may be present at any number of locations throughout the device.

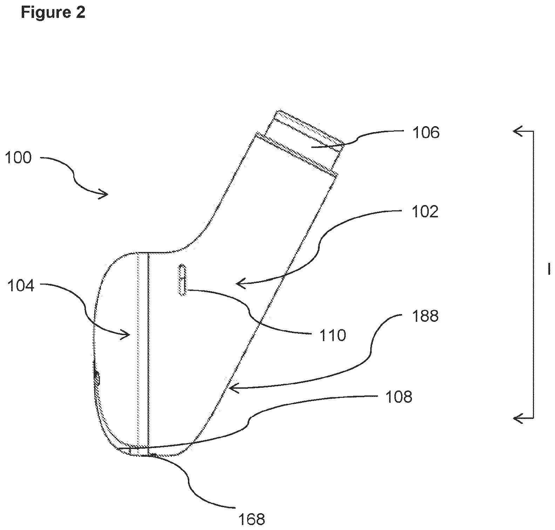

Shown in figure 2 is the mouth piece 122 is preferably covered by a mouth piece cover 108. The mouth piece 122 is shown as retracted and the mouth piece cover 108 is shown as being closed, prone to an exterior surface of the outlet member 104. Most preferably the mouth piece cover 108 is mounted to the inlet member, even more preferably by way of a hinge, and releasably engages with a surface of the outlet member 104. In a further set of embodiments the inlet member 104 is adapted to receive the MDI canister 106 and actuator 196 by having a complementary shaped recess the recess comprising an aperture and having a shape complementary a mouthpiece of the MDI canister 106 and actuator 196. Such an adaptation can be introduced at 188. Thus the mouthpiece of the MDI canister 106 and actuator 196 is received in the recess in fractional engagement where the MDI canister 106 and actuator 196 is readily removable whilst also capable of forming an at least partial seal when in use. In further embodiments the recess is shaped such that the MDI canister 106 and actuator 196 release of the medicament relative to the conduit substantially parallel to an axis of to the conduit. In this set of embodiments the saddle for actuating the MDI canister is associated with the MDI canister 106 and actuator 196.

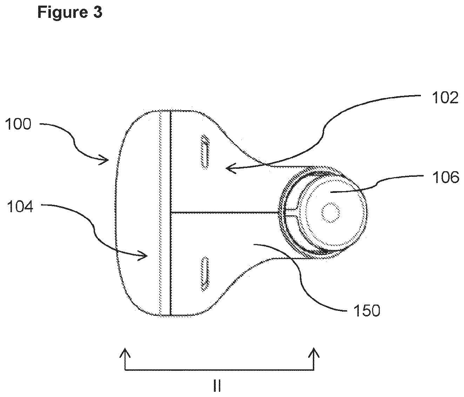

Figure 3 illustrates a top plan view of a preferred embodiment of the device when the support and conduit are in the collapsed state 100 according to one aspect of the invention. The view illustrates a preferred construction of the inlet member 104, being joined as two symmetrical halves 150, it is to be understood that this is a convenient means of assembly and as discussed the outlet member may take any form suitable to receive and actuate the canister 106 or MDI canister and actuator 196, and allow transfer of the medicament there through upon actuation.

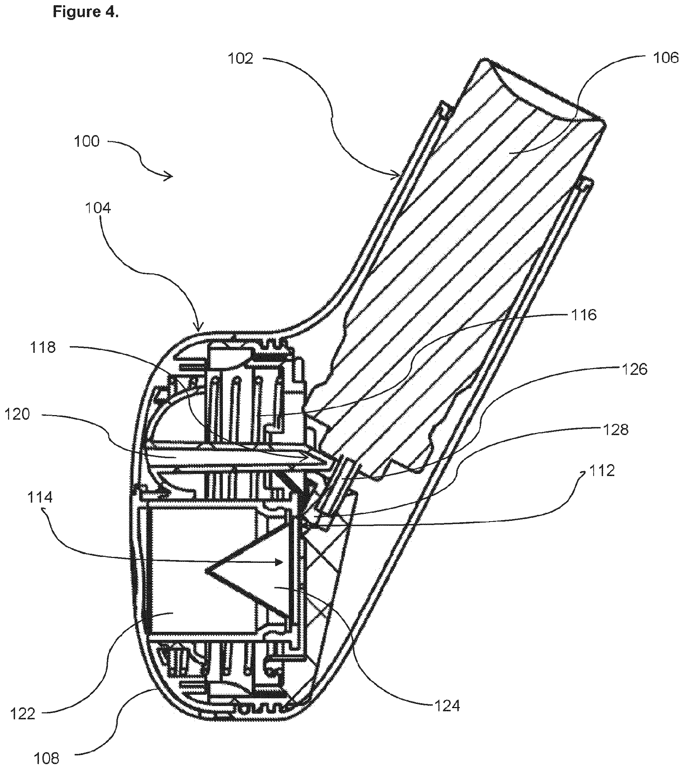

Figure 4 is a sectional side view through plane I of figure 2 of a preferred embodiment of the device in the collapsed state according to one aspect of the invention. In the collapsed state the inlet member 102 and outlet member 104 are proximate to each other, and as show are abutting. In the collapsed state the support 116 is shown as occupying less volume than in the expanded state. The pliable conduit 136, supported by the support 116 also occupies less volume and is compressed in the collapsed state. The support may take any form capable of supporting the outlet member 102 and pliable conduit 136, in a preferred embodiment the support is a spring 142.

Further shown in the sectional view is the canister 106 being received in the inlet member 102 by way of a saddle 128. The saddle accommodates a needle valve 126 of the canister 106. The skilled addressee will appreciate that the saddle 128 allows for depression of the canister 106, such that the needle 126 operates to open a valve contained inside thereof and dispense a metered dose of medicament into the device through an inlet 112 of the outlet member 102.

In some embodiments the device further comprises a lockout mechanism 118 for inhibiting operation of the canister 106 or MDI canister and actuator 196 when the support and conduit are in the contracted state and not inhibiting operation of the canister when the support and conduit are in the expanded state. The canister 106 contains the medicament and has a needle 128 having a valve for dispensing the medicament through. The needle valve opens upon depression of the canister 106 by engagement with the saddle 128. In the collapsed state, this process is inhibited by the lockout mechanism 118.

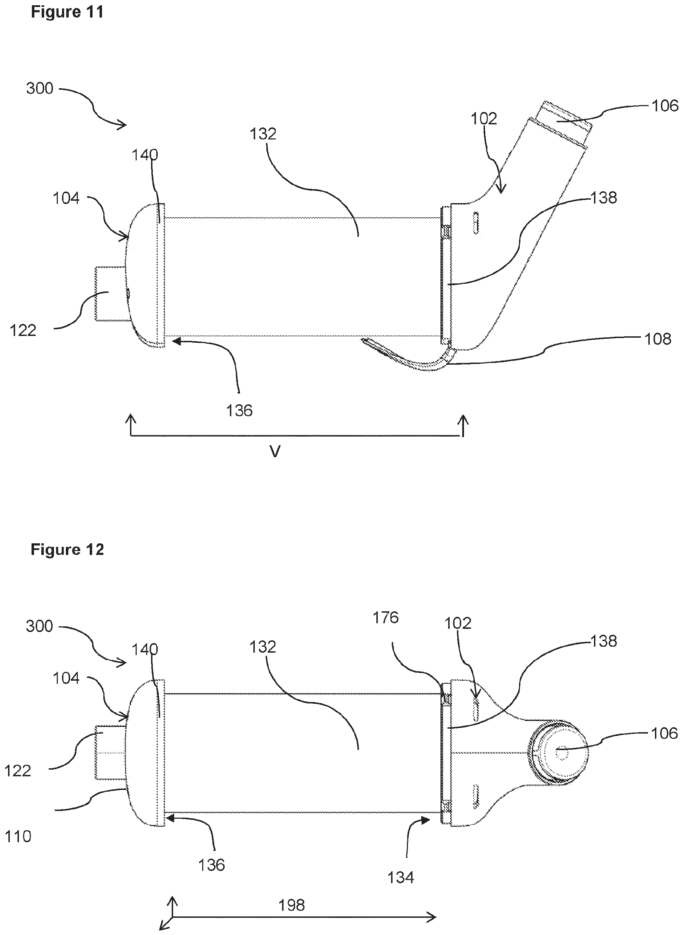

A preferred embodiment of the lockout mechanism 118 is shown in figure 4 as an elongate member 120 which extends in a manner parallel to axis parallel to the chamber (shown in figure 11 as arrow 198) and internal manner, from the outlet member 104. When the support and conduit of the device are in the collapsed state the elongate member 120 engages with the canister 106, preventing depression of the canister, and actuation of the needle valve and dispensation of the medicament. It should be appreciated that many arrangements of matter may serve to perform this function and the elongate member 120 servers only to exemplify one possible way of preforming the intended operation lockout mechanism 118.

Further shown in figure 4 is the mouth piece 122 shown as retracted, with the mouth piece cover 108 in a retaining position prone to the outlet member 104. In further embodiments of the invention, the mouth piece 122 is traversed by a one valve 124, preferably this valve may take the form of duck billed valve, which opens in response to negative by application of negative pressure through the mouth piece 122, but remains closed in response to application of positive pressure.

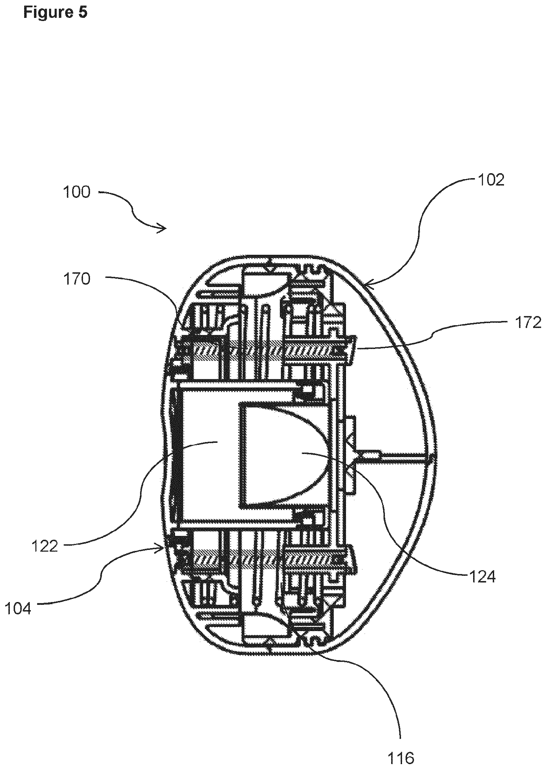

Figure 5 illustrates a sectional inverse plan view through plane II of figure 3 of a preferred embodiment of the device in the collapsed state according to one aspect of the invention. Shown here is the inlet member 102 proximate to the outlet member 104 in the collapsed state. Further shown is the support 116 in a collapse state. Also visible is the mouth piece 122 and one way valve 124 in a retracted position. In some embodiments the mouth piece 122 is biased to be in an extended position by a suitable means, preferable this may take the form of a spring, most preferably a set of springs 170, associated to the outlet member by any suitable means 170. Most preferably when the mouth piece 122 is retracted, the set of springs 170 is extended and under tension ready to act upon the mouth piece 122 on release of the mouth piece cover 108.

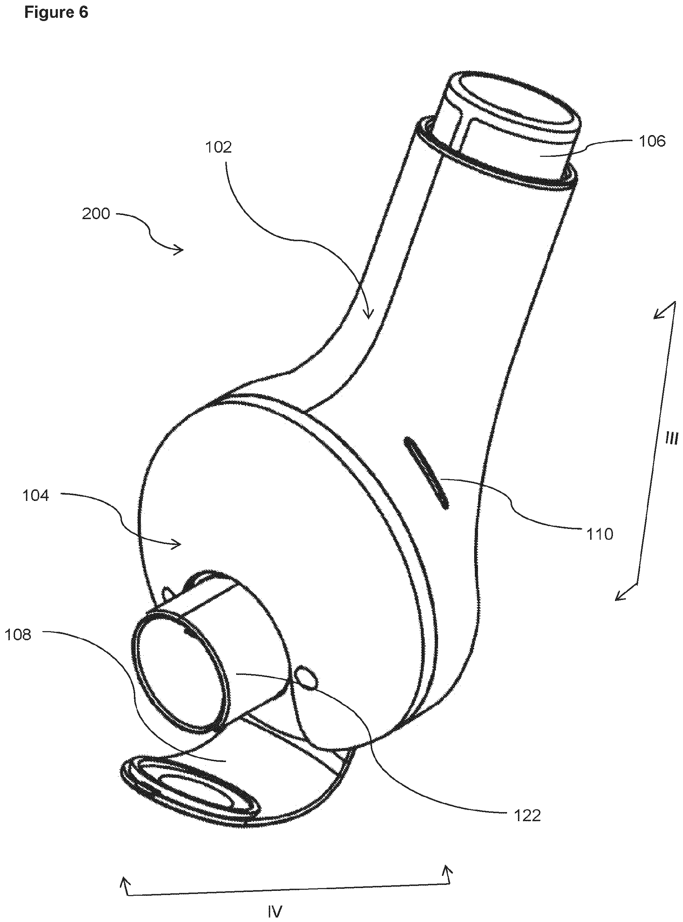

Figure 6 illustrates an isometric view of a preferred embodiment of the device when the support and conduit are in the collapsed state according to one aspect of the invention with mouthpiece 122 released and extended 200. Also shown is the mouth piece cover 108 released, most preferably the mouth piece is attached to the inlet ember 102 by way of a hinge.

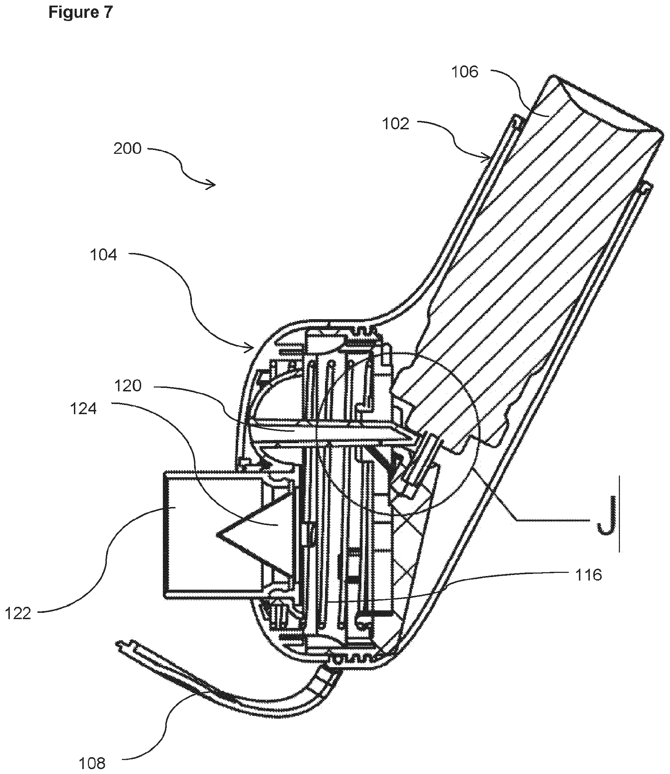

Figure 7 is a sectional side view through plane III of figure 6 of a preferred embodiment of the device in the collapsed state according to one aspect of the invention with mouthpiece released 200. This figure shows an embodiment of the invention where the mouth piece extended 122 and the mouth piece cover 108 is in a released position, however the lockout mechanism 118, shown in detail J remains engaged with the MDI 106 preventing actuation.

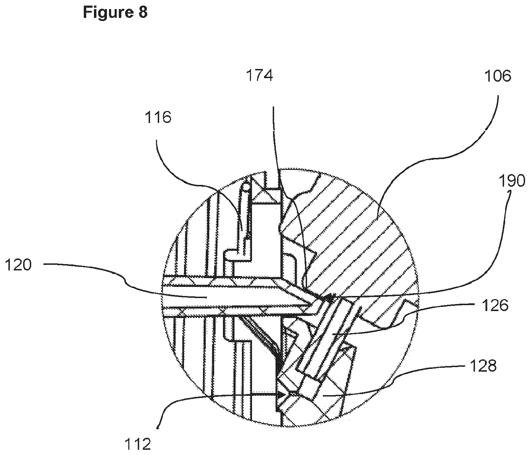

Figure 8 details an expanded view of detail "J", a preferred embodiment of lock out the mechanism when the conduit and support device is in a collapsed state according to one aspect of the invention. In the collapsed state, the lockout mechanism is characterised by an inability to actuate the MDI 106. In this embodiment the elongate member 120 attached to the outlet member 104 (not shown) engages with the MDI canister 106 or MDI canister and actuator 196, in this case a surface of a protrusion 174, prevention depression of the MDI canister 106 MDI canister and actuator 196 to act upon the saddle 112, thus preventing opening of the valve within the needle 126. This wedge like action is shown by arrow 190. Shown here the elongate member is performing the function of a wedge between the MDI canister 106 MDI canister and actuator 196, in this instance engagement occurs at the protrusion 174, and saddle 112. It will be appreciate that the lockout mechanism, such as elongate member 120, can act as a wedge when the inlet member 104 is adapted to receive an MDI canister and actuator 196, thus the saddle 128 is located in the MDI canister and actuator 196.

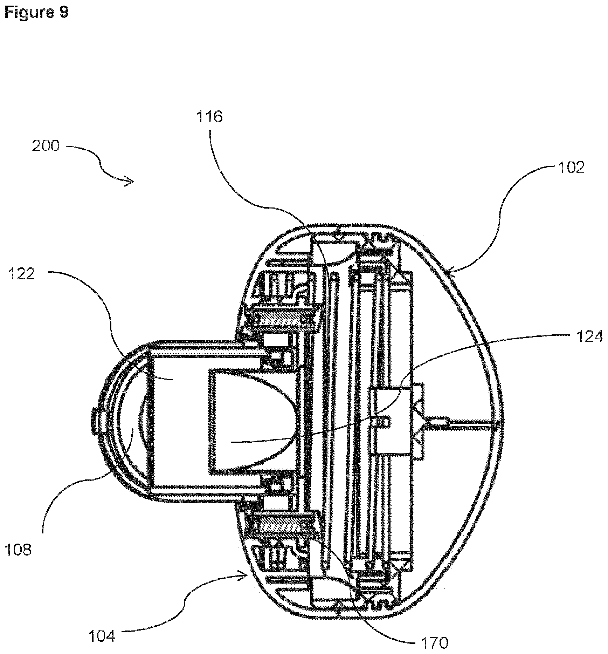

Figure 9 illustrates a sectional inverse plan view through a plane IV of figure 6 of a preferred embodiment when the support and conduit of the device in the collapsed state according to one aspect of the invention with mouthpiece released 200. Shown here, the mouth piece 122 is extended with the mouth piece cover 108 released. In a preferred embodiment the mouth piece 122 is biased to be in an extended position, as shown the extender, preferably a spring, most preferably a set of springs 170, is now therefore in contracted position acting upon the mouth piece 122 to extend it perpendicular to the surface of the outlet member 104.

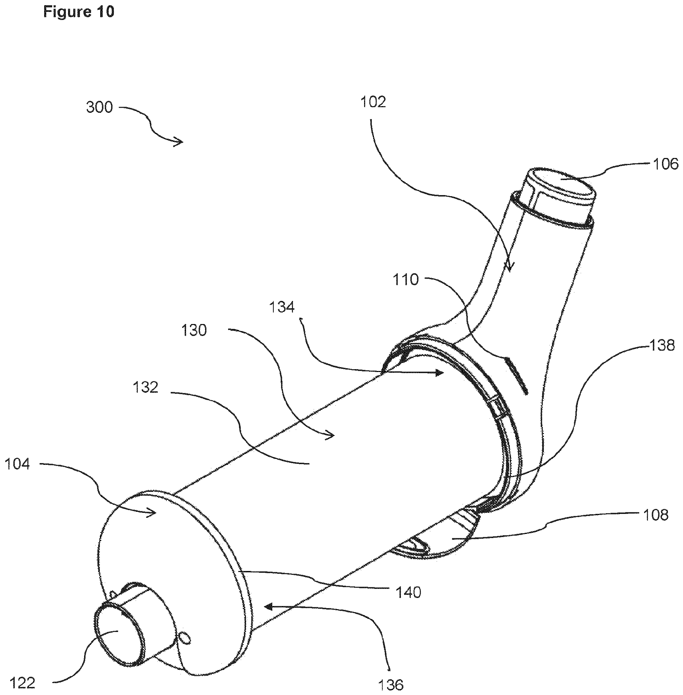

Figure 10 illustrates an isometric view of a preferred embodiment of the device when the support and conduit are in the expanded state 300 according to one aspect of the invention with mouthpiece released. In the expanded state, the pliable conduit 132 is expanded by the associated support (not visible) and the inlet member 102 is spaced form the outlet member 104. A chamber 130 is defied by the pliable conduit 132, having a proximal end 136 towards the outlet member 104 and a distal end 134 towards the inlet member 102. Further shown in figure 10 is the support 116 acting to support acting on the pliable conduit 132 in a taut manner and supporting the outlet member 104 without significant deformation of the conduit 130 and therefore the chamber 132.

Figure 11 displays a side view of a preferred embodiment of the device in the expanded state 300 according to one aspect of the invention with mouthpiece released. In some embodiments of the intervention the conduit 132 is tapered, preferably the taper is such that the circumference of the pliable conduit 132 and chamber 130 decreases towards the outlet member 104. In a preferred embodiment of the invention the inlet member 102 outlet member 104 and have first and second association portions 140 and 138 respectively for attaching the pliable conduit 132 and support 116. In figure 11 the chamber 130 is shown to be substantially linear along a longitudinal axis parallel to the chamber in the expanded state.

Figure 12 illustrates a plan view of a preferred embodiment of the device in the expanded state according to one aspect of the invention with mouthpiece released. Shown in both figures 12 and 11 is one embodiment for releasable engagement of the inlet 102 and outlet 104 members when the device is in the contracted state. As shown, a pair of is a resiliently deformable protrusions, 176, formed on the inlet member 102 capable of frictional engagement with the outlet member 102.

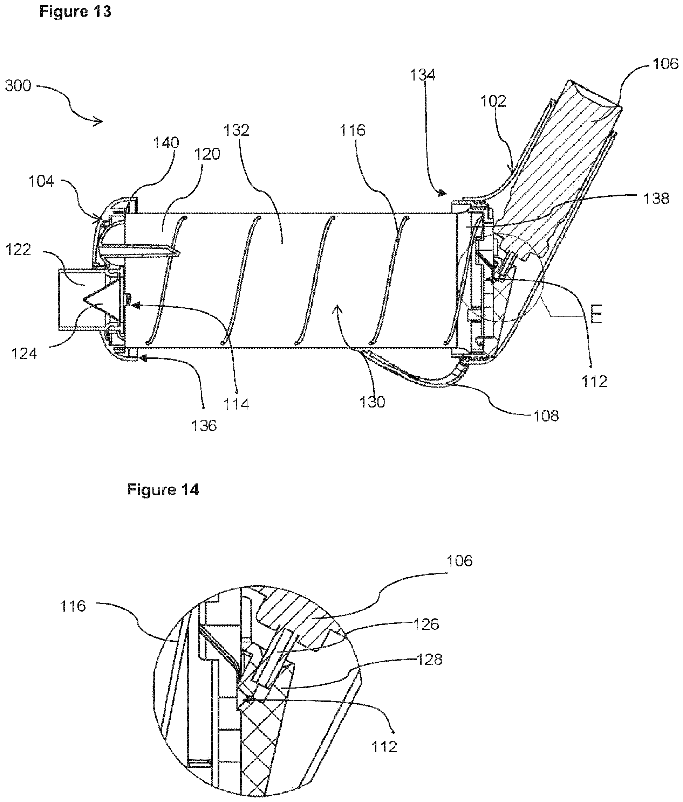

Figure 13 illustrates a sectional view through plane V of figure 11 of a preferred embodiment of the device when the conduit 130 and support 116 are in the expanded state 300 according to one aspect of the invention with mouthpiece released. Shown here is the support 116 supporting the pliable conduit 132 in the expanded state. In a preferred embodiment the support is a helical spring 142. Also shown is the perimeter of the chamber 130, the perimeter being defined by the pliable conduit 132, inlet member 102 and outlet member 104. Within the chamber is further defined a cavity volume 144 which according to a preferred aspect of the invention is around 200 mL. Shown at the proximal end 136 and at the distal end 134 of the pliable conduit 132 is the second association portion of the outlet member 140 and first association portion of the inlet member 138, in a preferred embodiment the support 116 and pliable conduit 132 are associated with thereon.

When the support 116 and conduit 132 of the device are in the expanded state, the lockout mechanism 118 does not prevent actuation of the MDI canister 106 or MDI canister and actuator 196 and release of the medicament therefrom. In a preferred embodiment an elongate member 120 can perform this action, and as shown in the expanded state the member 120 is no long engaging with the MDI canister 106 or MDI canister and actuator 196, therefore allowing depression and operation thereof.

Figure 14 details an expanded view of detail "E", a preferred embodiment of lockout the mechanism 118 when the conduit and support 116 is in an expanded state according to one aspect of the invention. Shown here is the MDI canister 106 or MDI canister and actuator 196is received in the saddle 128 by way of engagement with the needle 126. Without engagement of the elongate member 120, depression of the MDI canister 106 or MDI allows for actuation of the valve within the needle 128 and dispensation of the medicament through the inlet 112 into the chamber 130.

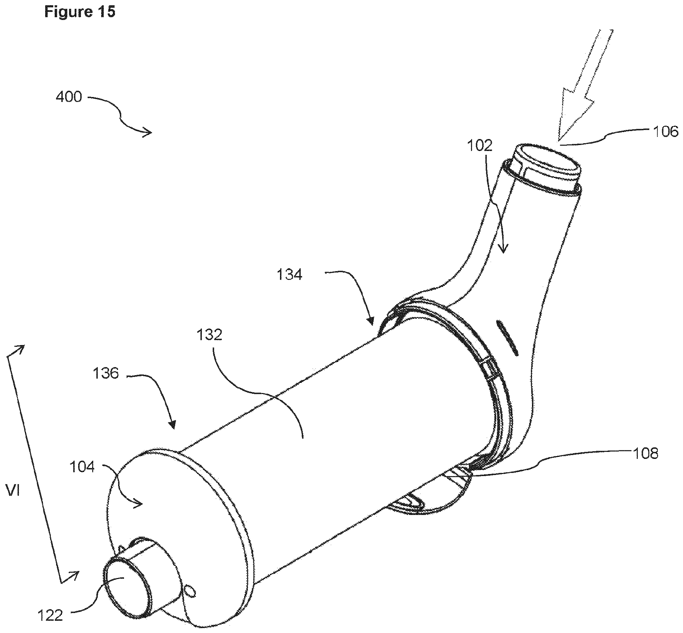

Figure 15 is an isometric view of a preferred embodiment expanded state according to one aspect of the invention with mouthpiece released with MDI depressed 400. Shown herein is the depressed MDI canister 106 or MDI canister and actuator 196, the arrow indicating the direction of force. Upon actuation of the MDI canister 106 or MDI canister and actuator 196, a metred dose of medicament is dispensed into the chamber 130 through an inlet 112 of the inlet member 102 according to one aspect of the invention. The medicament aerosol mixing with ambient air in the chamber 132, followed by inhalation through the mouth piece 122 of the outlet member 104 by the patient. Subsequently air is interred into the chamber 132 through the at least one vent 110, further carrying and mixing the metered dose of medicament to equalise the pressure caused by inhalation through the use of negative thoracic pressure.

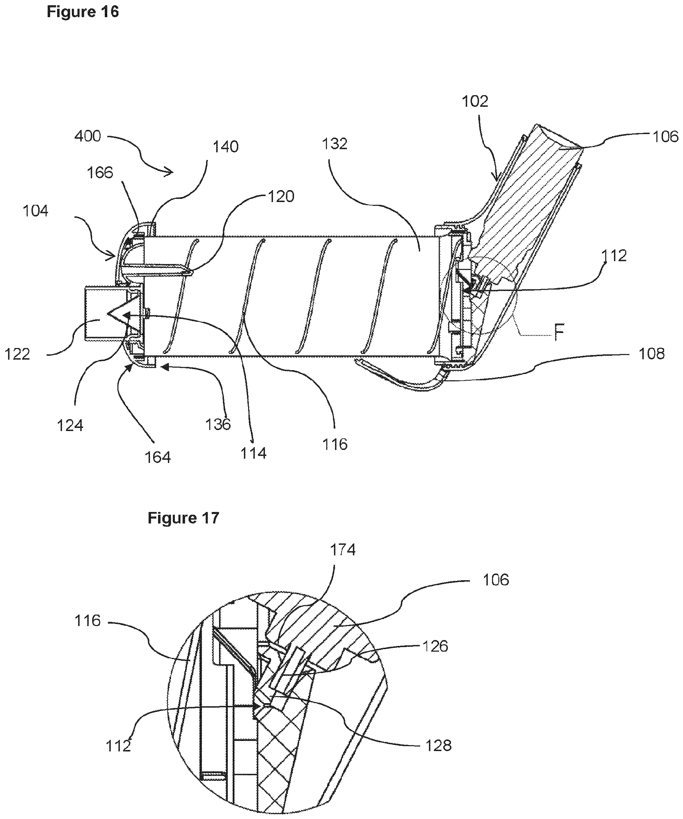

Figure 16 is a sectional side view along plane VI of figure 15 of a preferred embodiment expanded state according to one aspect of the invention with mouthpiece released with MDI depressed 400. Shown here is the inlet 122 of the inlet member 102 which medicament dispensed from the MDI canister 106 or MDI canister and actuator 196 travels through to enter into the camber 130 as defined. The metered dose of medicament mixes with air within the cavity volume 144, before inhalation by the patient. The patient performs inhalation by use of negative thoracic upon mouth piece 122, which in turn opens the duck billed valve 124 enabling the dose of medicament within the chamber 130 to exit through the outlet 144 of the outlet member 104 into the patient's respiratory system. As discussed, the negative pressure thus produced within the chamber 130 can then be equalised by intrusion of air through a vent 110, as stated the vent is preferably a one way vent to prevent escape of medicament prior to inhalation. Any expired air into the mouth piece 122 can be directed out of the device through any number out outflow vents 184. Shown here the MDI canister 106 is received in the saddle 128 by way of engagement with the needle 126. Without the elongate member 120, depression of the MDI canister 106 allows for actuation of the valve within the needle 128 and dispensation of the medicament through the inlet 112 into the chamber 130.

Figure 17 is an expanded view of detail "F", a preferred embodiment of lock out the mechanism 118 when the conduit and support are in the expanded state with the MDI canister 106 or MDI canister and actuator 196 depressed according to one aspect of the invention. Shown in detail F is the MDI canister 106 or MDI canister and actuator 196 as actuated, with the lockout mechanism 118 and not inhibiting depression and therefore actuation of the MDI canister 106. In this particular embodiment depression of the MDI 106 leads to actuation of a valve within the needle 126 by engagement with the saddle 128. The body of the MD canister slides over the needle 128, until the protrusion 174 abuts the saddle 128 causing opening of the valve and dispensation of the medicament thought the inlet 112 into the device.

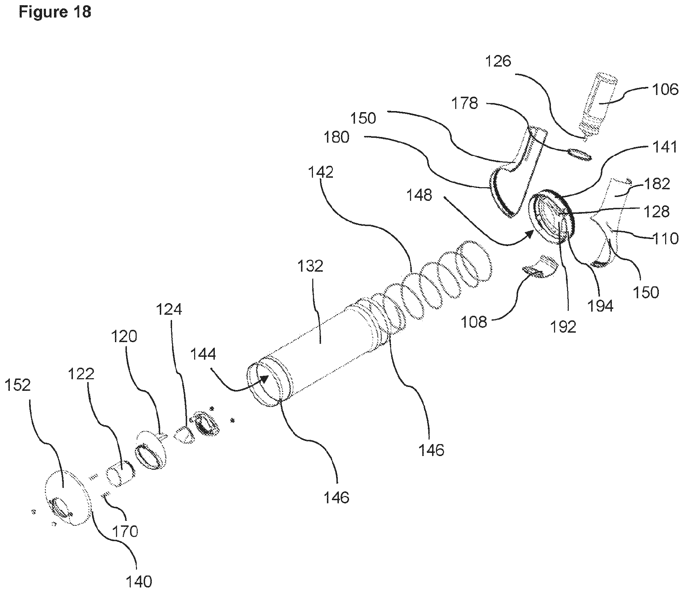

Figure 18 is an isometric, exploded view of a preferred embodiment of the device according to one aspect of the invention. Shown here is the inlet member 102 comprising in a preferred embodiment a circular mount 148 having the saddle arrangement 128 and alternate support and conduit association point, the load bearing section 141. Also shown in a preferred arrangement are the two halves 150 forming a tube shaped appendage 182 to receive the MDI canister 106 or MDI canister and actuator 196 and forming the circular portion 180 to enclose the circular mount 148. In some embodiments the vent 110 is placed on the inlet member. In some embodiments the MDI canister 106 or MDI canister and actuator 196 is held in place using an annular seal 178. The mouth piece cover 108 is preferably attached by way of a hinge. In some embodiments, the inlet member 102 has a first association portion 138 for attaching the distal end of the support 116 and conduit 132. Most preferably the support is a spring 142, and both spring 142 and the pliable conduit 132 are associated with to afirst association portion 138 at the distal end 134 by way of a frictionally engaging o-ring 146. Figure 18 further shows an embodiment of the mount 148 comprising radial spokes 192 and outer rim 194.

The pliable conduit 132, inlet member 102 and outlet member 104 define a chamber 130 having a cavity volume 144 in the expanded state. In the expanded state the pliable conduit 132 and outlet member 104 are supported by the support 116, in a preferred embodiment the support is a spring 142. In some embodiments, the proximal end 136 of the conduit and 132 and support 116 are associated with to first association portion 138 the outlet member 104 by way of a frictionally engaging o-ring 146. The support 116 can be associated with the conduit by any means, for example, by tension of the material and spring, the spring can be interwoven within the conduit, a plurality of attaches, such as o-rings associated with the conduit and the spring passing there through can be used, the spring can also be associated with the support by way of adhesive.

The outlet member 102 is configured to allow a patient to inhale medicament from the device, in a preferred embodiment a mouth piece 122 is provided to perform this function. Even more preferably the mouth piece 122 is traversed by a one way duck bill valve 124, configured to open on application of negative thoracic pressure. Even more preferably the mouth piece 122 is configured to extend out of the outlet member 102. In the embodiment shown, a basing means, in a preferred embodiment a set of springs 170 biases the mouth piece 122 to extend from the outlet member 102, but is held prone to the outer surface of the outlet member 102 by a cover 108. Also shown, is a preferred embodiment of the lockout mechanism 118, taking the form of an elongate member 120, which extends into the chamber 130 to engage with the MDI canister 106 or MDI to prevent actuation when in the contracted state, and not inhibit actuation when in the expanded state.

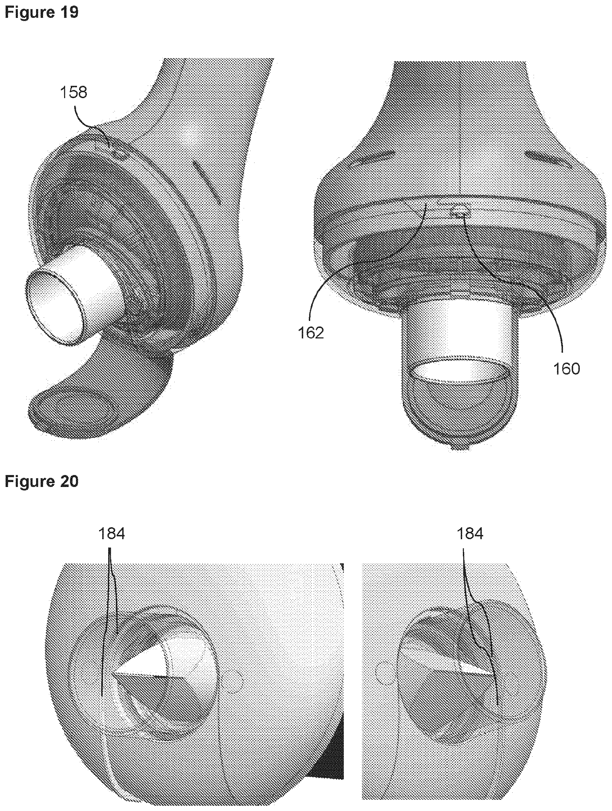

Figure 19 shows an isometric view and a plan view of a preferred embodiment of the device in a contracted state, with the mouth piece open, showing bayonet fittings according to one aspect of the invention. Shown in this figure is a preferred embodiment of a releasably engaging means when the device is in a contracted state and the outlet member 104 and inlet member 102 are proximal to each other. Preferably, the engagements means takes that from of a bayonet type fitting 158 comprised of a lug 160 and radially traversing recess 162 to receive the lug. Most preferably the three sets of lugs 160 and corresponding recesses 162 are arranged at 120° positions around a circumference of the device as shown in the figures.

Figure 20 shows a perspective view of a preferred embodiment when the conduit 132 and support 116 are in the contracted state, with the mouth piece extended 122, showing the preferred arrangement of the outflow vents 184. The mouth piece 122 further comprises at least one outflow vent 184 directing any expired air into the surrounding atmosphere. It will be appreciated that the outflow vent 184 may be situated at any suitable location on the device, and any suitable number of vents may be used. Preferably the outflow vent 184 traverses from the cavity in between the mouth piece and one way valve to an exterior of the device. Most preferably the device comprises four outflow vents 184 radially dispersed around the mouth piece at 90° intervals.

### System for Respiratory Drug Delivery

The present invention relates to a collapsible inhaler for use with an MDI dispenser, and further to systems of respiratory drug delivery. The device in combination with an MDI dispenser provides in a set of embodiments a system for respiratory delivery of a medicament, the system comprising an inhaler as previously described and an dispense, wherein the dispenser contains a medicament composition comprising a pharmaceutically active agent.

An MDI dispenser can comprise a MDI canister 106 or an MDI canister and actuator 196. The canister 106 containing medicament, a metering valve which releases a metered dose of medicament upon actuation, and a needle 126 which allows the medicament to exit the canister, often as an aerosol. Actuation is achieved by depression of the canister whilst the needle is held in a suitable means, such as the saddle arrangement 128 as shown previously, or where the saddle forms part of an MDI actuator, which results in the canister sliding over the needle 126 and opening of the calibrated valve to release the metered dose of medicament. Often the canister is pressurised such that the medicament is ejected under pressure through the needle value and dispersed as an aerosol which has the advantage of having a high surface area which encourages adsorption in to the patient. The chamber 132 provided by the conduit 132 and support 116 in the expanded state provides a cavity volume 144 within which the aerosol of medicament expands into. The patient then inhales the volume of expanded medicament. The volume 144 allows for the initially concentrated medicament aerosol to expand after ejection from the MDI canister 106 or MDI canister and actuator 196. The patient may then apply negative thoracic pressure and inhale the medicament to act upon or be absorbed by the pulmonary system. The use of a chamber 132 encourages a patient to inhale in a more consistent, deep and regular manner.

The system results in a more efficacious, consistent and less wasteful way of delivering pharmaceutically active agents through the respiratory system. In the expanded state, the chamber further slows the ejected medicament resulting in decreased deposition of medication in the oropharynx whose effect is both more reliable pulmonary delivery as well as decreased risk of side effects, such as hoarse voice. The system also negates the requirement for coordination of the MDI canister 106 MDI or canister and actuator 196 actuation with inhalation, which especially useful in young or unwell patients. In the expanded state, the chamber 130 further slows the ejected medicament resulting in decreased deposition of medication in the oropharynx whose effect is both more reliable pulmonary delivery as well as decreased risk of side effects, such as hoarse voice.

In some embodiments, the system further comprises the lockout 116 mechanism, as previously discussed, operation of the MDI dispenser is inhibited when the support is in the contracted state and operation of the MDI dispenser not inhibited when the support is in the expanded state.

In a further set of embodiments the system is adapted for paediatric use. Such adaptation may take the form of a cavity volume 144 adapted for paediatric lung capacity, in a preferred embodiment the volume is about 200 ml. In further embodiments the mouth piece 122 is adapted to fit a child's mouth size. In yet further embodiments the support 116, preferably a spring 142 has a tension not unduly high to be used by a child. Furthermore the adjustable support and conduit is user friendly which enables children of most ages to extend and collapse the device. In some embodiments, the outlet member further comprises a grip which may be formed of an over-moulded rubber or silicone grip. The grip may be incorporated into or mounted on the outlet member. This aids in the user gripping the outer during the twisting motion of the bayonet locking engagement which may be especially useful for young and/or unwell patients.

In further embodiments of the invention the system comprises an MDI dispsnser contains a composition comprising a pharmaceutically active agent selected from the list comprising, short-acting beta-adrenergic agonists, corticosteroids, tocolytics, long-acting beta-adrenergic agonist leukotriene inhibitors, mast cell stabilizers, phosphodiesterase 4 (PDE4) inhibitors, inhaled magnesium sulphate or mixtures thereof. Most preferably the system comprises and MDI dispenser comprising a pharmaceutically active agent for delivering respiratory drugs for the prophylaxis and/or treatment of asthma and related disorders characterized by symptoms such as reversible airflow obstruction, and/or bronchospasm. Such disorders may further include chronic obstructive pulmonary disease (COPD) and transient bronchospasm relating from allergens as might be experienced during thunderstorm asthma events.

In some embodiments the system is adapted to prevent adherence of the pharmaceutically active agent. Such adaptations include, the interior of the conduit 132 being smooth, featureless or continuous inner surface forming part of the chamber 130 which prevents physical adherence of the pharmaceutically active agent in discontinuities in the pliable conduit 132. The surface may also have a polish, gloss or be coated by an anti-static or non-stick material preventing adherence of the pharmaceutically active agent. Most preferably all surfaces exposed to the medicament containing the pharmaceutically active agent are adapted as discussed to prevent adherence, including the support 116, outlet member 104, inlet member 102, duck billed valve 124 and the mouth piece 122.

In yet further embodiments the medicament is comprised of the pharmaceutically active agent, various excipients, propellants and mixtures thereof.

A pharmaceutically active agent is to be understood as meaning the ingredient in a medicament that is biologically active. The active ingredient may have many active constituents whether defined or otherwise and may be a pharmaceutically acceptable salt or other acceptable formulation.

An excipient is to be understood as pharmaceutically inactive substance that serves to aid in the drug delivery, enchase shelf life, aid in solubility, facilitate flowability, reduce viscosity or otherwise enhance the properties of pharmaceutically active agent.

Common excipients include a pH regulator, a chelating agent, a tonicity adjusting agent, a vehicle, a solvent, a sweetener, a buffering agent, a preservative, or mixtures thereof.

A propellant is to be understood as compressible substance which is not substantially toxic and preferably does not cause undue environmental harm. In preferred embodiments the propellant is a fluoroalkane. Common fluoroalkanes include 1,1,1,2-tetrafluoroethane. In the compositions to be delivered with an MDI dispenser the propellant is preferably a hydrofluorocarbon selected from the group of HFA 134a, HFA CF₃CHFCF₃, also known as HFA 227, HFC 227 or 1,1,1,2,3,3,3- heptafluoropropane.

The following medicaments are provided by way of example of medicaments that may be administered by the system as described. These are exemplary in nature and not meant as a limitation to what may be contained in the MDI dispenser.

In one embodiment the system comprises an MDI dispenser containing a short acting bronchodilator. Short acting bronchodilators act upon the lungs to open air ways to improve breathing. Examples of a short acting bronchodilator may be selected from the list comprising albuterol sold under the trade name Vospire ER, levalbuterol sold under the trade name Xopenex, ipratropium sold under the trade name Atrovent, albuterol/ipratropium sold under the trade name Combivent, or mixtures thereof. The MDI disperser may further comprise theophylline.

In another embodiment the system comprises an MDI dispenser containing a long acting bronchodilators. Long acting bronchodilators also act upon the lungs to open air ways to improve breathing, but generally operate for a longer period of time. Examples of long acting bronchodilators may be selected from the list comprising aclidinium sold under the trade name Tudorza, arformoterol sold under the trade name Tudorza, arformoterol sold under the trade name Brovana, formoterol sold under the trade name Foradil and Perforomist, glycopyrrolate sold under the trade name Seebri and Neohaler, indacaterol sold under the trade name Arcapta, olodaterol sold under the trade name Striverdi and Respimat, salmeterol sold under the trade name Serevent, tiotropium sold under the trade name Spiriva, umeclidinium sold under the trade name Incruse Ellipta and Brovana, or mixures thereof.

In another embodiment the system comprises an MDI dispenser containing a Corticosteroid. Corticosteroids reduce inflammation in the lungs and airways to improve breathing. Examples of Corticosteroids, often used for the treatment of COPD and/or asthma may be selected from the list comprising Fluticasone sold under the trade name Flovent, Budesonide sold under the trade name Pulmicort, and Prednisolone.

In another embodiment the system comprises an MDI dispenser containing a combination of drugs. Examples of common combinations may be selected from the list comprising glycopyrrolate and formoterol sold under the trade name Bevespi and Aerosphere, glycopyrrolate and indacaterol sold under the trade name Utibron amd Neohaler), tiotropium and olodaterol sold under the trade name Stiolto and Respimat, umeclidinium and vilanterol sold under the trade name Anoro and Ellipta, budesonide and formoterol sold under the trade name Symbicort, fluticasone amd salmeterol sold under the trade name Advair, fluticasone and vilanterol sold under the trade name Breo and Ellipta.

In another embodiment the system comprises an MDI dispenser containing Leukotriene receptor antagonists, which may be selected from the list comprising montelukast and zafirlukast. The MDI may also comprise a mast cell stabiliser such as cromolyn sodium. In further embodiments the MDI dispenser can comprise phosphodiesterase 4 (PDE4) inhibitors such as Cilomilast, Ibudilast, Roflumilast or mixtures thereof

### Method of Respiratory Drug Delivery which is not claimed

According to a further aspect there is provided a method of respiratory administration of a medicament to a subject in need thereof the medicament comprises a composition comprising a pharmaceutically active agent. The method comprising providing the inhaler of as described, inserting an MDI dispenser into the inlet member 102; operating the MDI dispenser to dispense a metred dose of the medicament through the inlet 112 of the inlet member 102 into the chamber 130, wherein the medicament is dispersed within the cavity volume 144 of the chamber 130; and inhaling the medicament through the outlet 144 out the outlet member 104. The medicament can be dispersed, often by action of propellant, and mix with ambient air of the volume 144 of the chamber 130 to from a more homogenised aerosol, before inhalation. Furthermore, inhalation by negative thoracic pressure aids in mixing of the medicament. In some embodiments the inhaler further comprises vents 110 which allow passage of the air, preferably one-way into the device further aiding in mixing of the medicament.

The use of a chamber 132 encourages a patient to inhale in a more consistent, deep and regular manner. The method resulting in a more efficacious, consistent and less wasteful way of delivering pharmaceutically active agents through the respiratory system. Operation of the MDI dispenser is by depression and leads to actuation of a valve within the needle 126 by engagement with the saddle 128. The MDI canister 106 slides over the needle 128 until opening of the value contained therein leading to dispensation of a metered dose of medicament. Generally the composition comprises a propellant which propels the metered dose of medicament from within the canister.

In some embodiments the protrusion 174 abuts the saddle 128 during depression leading to dispensation of the medicament thought the inlet 112 into the chamber 130 of the inhaler and to mix with ambient air within the cavity volume 144. In the expanded state, the chamber 130 further slows the ejected medicament resulting in decreased deposition of medication in the oropharynx whose effect is both more reliable pulmonary delivery as well as decreased risk of side effects, such as hoarse voice. The method further negates the requirement for coordination of the MDI dispenser actuation with inhalation, which especially useful in young or unwell patients. In the expanded state, the chamber 130 further slows the ejected medicament resulting in decreased deposition of medication in the oropharynx whose effect is both more reliable pulmonary delivery as well as decreased risk of side effects, such as hoarse voice.

In a further set of examples, operating of the MDI dispenser 106 in the method is inhibited when the support 116 and conduit 132 is in the contracted state and operation of the MDI dispenser 106 not inhibited when the support 116 and conduit 132 is in the expanded state. In some examples the method further comprises a step of operating the inhaler such that the lockout mechanism 116 does not prevent actuation of the MDI dispenser 106. The step comprises operating the inhaler such that conduct 132 and support 116 are in the extended state allowing for operation of the MDI dispenser 106, andthe elongate member 120 does not engage the MDI dispenser 106 allowing for operation thereof in the expanded state. Even more preferably operating the inhaler is such that the realisably engaging apparatus, preferably the bayonette fitting comprising lugs 160 and recesses 162, is released allowing for extending of the support 116 and conduit 132 into the extended state, disengaging the elongate member 120 with the MDI 106 allowing operation thereof.

In a further set of examples the method is adapted for paediatric use. Such adaptation may take the form of a chamber volume 144 adapted for paediatric lung capacity, in a preferred embodiment the volume is about 200 ml. In further embodiments the mouth piece 122 is adapted to fit a child's mouth size. In yet further embodiments the support 116, preferably a spring 142 has a tension not unduly high to be used by a child.

In yet further embodiments the medicament is comprised of the pharmaceutically active agent, various excipients, propellants and mixtures thereof.

A "pharmaceutically active agent" is to be understood as meaning the ingredient in a medicament that is biologically active. The active ingredient may have many active constituents whether defined or otherwise and may be a pharmaceutically acceptable salt or other acceptable formulation.

An excipient is to be understood as pharmaceutically inactive substance that serves to aid in the drug delivery, enchase shelf life, aid in solubility, facilitate flowability, reduce viscosity or otherwise enhance the properties of pharmaceutically active agent.

Common excipients include a pH regulator, a chelating agent, a tonicity adjusting agent, a vehicle, a solvent a sweetener, a buffering agent, a preservative, or mixtures thereof.

A propellant is to be understood as compressible substance which is not substantially toxic and preferably does not cause undue environmental harm. In preferred embodiments the propellant is a fluoroalkane. Common fluoroalkanes include 1,1,1,2-tetrafluoroethane. In the compositions to be delivered with an MDI dispenser the propellant is preferably a hydrofluorocarbon selected from the group of HFA 134a, HFA CF₃CHFCF₃, also known as HFA 227, HFC 227 or 1,1,1,2,3,3,3- heptafluoropropane.

In further examples the method comprises using an MDI comprises a pharmaceutically active agent selected from the list comprising, short-acting beta-adrenergic agonists, corticosteroids, tocolytics, long-acting beta- adrenergic agonist leukotriene inhibitors, mast cell stabilizers, phosphodiesterase 4 (PDE4) inhibitors, inhaled magnesium sulphate or mixtures thereof. Most preferably the method is used for delivering respiratory drugs for the prophylaxis and/or treatment of asthma and related disorders characterized by symptoms such as reversible airflow obstruction, and/or bronchospasm. Such disorders may further include chronic obstructive pulmonary disease (COPD) and transient bronchospasm relating from allergens as might be experienced during thunderstorm asthma events.

The following pharmaceutically active agents are provided by way of example of medicaments that may be administered by the method as described. These are exemplary in nature and not meant as a limitation to what may be contained in the MDI dispenser.

In one example the method uses an MDI dispenser containing a short acting bronchodilator. Short acting bronchodilators act upon the lungs to open air ways to improve breathing. Examples of a short acting bronchodilator may be selected from the list comprising albuterol sold under the trade name Vospire ER, levalbuterol sold under the trade name Xopenex, ipratropium sold under the trade name Atrovent, albuterol/ipratropium sold under the trade name Combivent, or mixtures thereof. The MDI disperser may further comprise theophylline.

In another example the method uses an MDI dispenser containing a long acting bronchodilators. Long acting bronchodilators also act upon the lungs to open air ways to improve breathing, but generally operate for a longer period of time.
Examples of long acting bronchodilators may be selected from the list comprising aclidinium sold under the trade name Tudorza, arformoterol sold under the trade name Tudorza, arformoterol sold under the trade name Brovana, formoterol sold under the trade name Foradil and Perforomist, glycopyrrolate sold under the trade name Seebri and Neohaler, indacaterol sold under the trade name Arcapta, olodaterol sold under the trade name Striverdi and Respimat, salmeterol sold under the trade name Serevent, tiotropium sold under the trade name Spiriva, umeclidinium sold under the trade name Incruse, Ellipta and Brovana, or mixures thereof.

In another example the method uses an MDI dispenser containing a Corticosteroid. Corticosteroids reduce inflammation in the lungs and airways to improve breathing. Examples of Corticosteroids, often used for the treatment of COPD and/or asthma may be selected from the list comprising Fluticasone sold under the trade name Flovent, Budesonide sold under the trade name Pulmicort, and Prednisolone.

In another example the method uses an MDI dispenser containing a combination of pharmaceutically active agents. Examples of common combinations may be selected from the list comprising glycopyrrolate and formoterol sold under the trade name Bevespi and Aerosphere, glycopyrrolate and indacaterol sold under the trade name Utibron amd Neohaler), tiotropium and olodaterol sold under the trade name Stiolto and Respimat, umeclidinium and vilanterol sold under the trade name Anoro and Ellipta), budesonide and formoterol sold under the trade name Symbicort, fluticasone amd salmeterol sold under the trade name Advair, fluticasone and vilanterol sold under the trade name Breo and Ellipta.

In another example the system comprises an MDI dispenser containing Leukotriene receptor antagonists, which may be selected from the list comprising montelukast and zafirlukast. The MDI may also comprise a mast cell stabiliser such as cromolyn sodium. In further embodiments the MDI may comprise phosphodiesterase 4 (PDE4) inhibitors such as Cilomilast, Ibudilast, Roflumilast or mixtures thereof.

In a further set of examples there is provided a method of treating or prophylaxis of respiratory diseases to a subject in need thereof comprising providing the inhaler as previously described, inserting an MDI dispenser into the inlet member 102; operating the MDI dispenser to dispense a metred dose of the medicament through the inlet 112 of the inlet member 102 into the chamber 130, wherein the medicament is dispersed within the cavity volume 144 of the chamber 130; and inhaling the medicament through the outlet 144 out the outlet member 104.

Preferably the respiratory disease is selected from asthma, transient bronchospasm relating from allergens as might be experienced during thunderstorm asthma events and/or COPD. Even more preferably the pharmaceutically active agent. Short acting bronchodilators act upon the lungs to open air ways to improve breathing. In further examples of the invention the method comprises using an MDI comprises a pharmaceutically active agent selected from the list comprising, short-acting beta-adrenergic agonists, corticosteroids, tocolytics, long-acting beta- adrenergic agonist leukotriene inhibitors, mast cell stabilizers, phosphodiesterase 4 (PDE4) inhibitors, inhaled magnesium sulphate or mixtures thereof. Most preferably the system is used for delivering respiratory drugs for the prophylaxis and/or treatment of asthma and related disorders characterized by symptoms such as reversible airflow obstruction, and/or bronchospasm. Such disorders may further include chronic obstructive pulmonary disease (COPD).

Examples of a short acting bronchodilator may be selected from the list comprising albuterol sold under the trade name Vospire ER, levalbuterol sold under the trade name Xopenex, ipratropium sold under the trade name Atrovent, albuterol/ipratropium sold under the trade name Combivent, or mixtures thereof. The MDI disperser may further comprise theophylline.

In another example the method uses an MDI dispenser containing a long acting bronchodilators. Long acting bronchodilators also act upon the lungs to open air ways to improve breathing, but generally operate for a longer period of time. Examples of long acting bronchodilators may be selected from the list comprising aclidinium sold under the trade name Tudorza, arformoterol sold under the trade name Tudorza, arformoterol sold under the trade name Brovana, formoterol sold under the trade name Foradil and Perforomist, glycopyrrolate sold under the trade name Seebri and Neohaler, indacaterol sold under the trade name Arcapta, olodaterol sold under the trade name Striverdi and Respimat, salmeterol sold under the trade name Serevent, tiotropium sold under the trade name Spiriva, umeclidinium sold under the trade name Incruse Ellipta and Brovana, or mixures thereof.

In another example the method uses an MDI dispenser containing a Corticosteroid. Corticosteroids reduce inflammation in the lungs and airways to improve breathing. Examples of Corticosteroids, often used for the treatment of COPD and/or asthma may be selected from the list comprising Fluticasone sold under the trade name Flovent, Budesonide sold under the trade name Pulmicort, and Prednisolone.

In another example the method uses an MDI dispenser containing a combination of drugs. Examples of common combinations may be selected from the list comprising glycopyrrolate and formoterol sold under the trade name Bevespi and Aerosphere, glycopyrrolate and indacaterol sold under the trade name Utibron amd Neohaler), tiotropium and olodaterol sold under the trade name Stiolto and Respimat, umeclidinium and vilanterol sold under the trade name Anoro and Ellipta), budesonide and formoterol sold under the trade name Symbicort, fluticasone amd salmeterol sold under the trade name Advair, fluticasone and vilanterol sold under the trade name Breo and Ellipta.

In another example the method uses an MDI dispenser containing Leukotriene receptor antagonists, which may be selected from the list comprising montelukast and zafirlukast. The MDI dispenser may also comprise a mast cell stabiliser such as cromolyn sodium. Further embodiments the MDI dispenser may comprise phosphodiesterase 4 (PDE4) inhibitors such as Cilomilast, Ibudilast, Roflumilast or mixtures thereof.

The skilled artisan will appreciate that the device, system and methods as previously described provides a convenient means of delivering respiratory drugs. When expanded the patient has increased reliability of dosage delivered into their pulmonary system, consistent, extended and deeper inhalation and overall reliability of respiratory drug delivery efficacy. In the expanded state, the chamber further slows the ejected medicament resulting in decreased deposition of medication in the oropharynx whose effect is both more reliable pulmonary delivery as well as decreased risk of side effects, such as hoarse voice. As discussed, the support conveniently supports the pliable conduit and the outlet member in the expanded state conveniently allowing the patient to hold and actuate the device using the inlet member. The pliability of the supported conduit enables collapsing of the device to decrease in volume providing the contracted state. Preferably the support is such that in the expanded state the pliable conduit is held taut and such that the weight of the outlet member is supported without substantial deformation of the chamber. The conduit, outlet member and inlet member define a chamber, having a cavity volume wherein the medicament mixes. The pliable conduit may be formed of any deformable material, preferably a durable plastic. Most preferably the combination of support and taut conduit allows inhalation of the medicament without substantial collapse of the camber.

The inhaler, system and method further comprise a lockout mechanism for inhibiting operation of the MDI dispenser when the support and conduit are in the contracted state. The mechanism does not inhibit operation of the MDI dispenser when the support is in the expanded state. The MDI dispenser operates by having a body containing the medicament and a needle valve for dispensing the medicament there through, the valve opening upon depression of the MDI dispenser through engagement with a saddle. The lockout mechanism prevents depression of the MDI dispenser when the support and conduit is in the contracted state by engaging with the MDI dispenser and preventing actuation.

The lockout mechanism provides the advantage of inhibiting actuation of MDI dispenser whilst the device is in the contracted state, therefore coercing the patient to use the device in the expanded state and obtain the advantages provided by the expanded state as discussed. Furthermore, the lockout mechanism prevents unintended actuation of the MDI dispenser during storage, transport and manipulation reducing wastage of medicament and wear to the MDI dispenser.

## Claims

1. A collapsible inhaler (100) for use with a metered dose inhaler (MDI) dispenser that is operable to dispense a metered dose of medicament therefrom, the inhaler (100) comprising:
an inlet member (102) being adapted to receive an MDI dispenser and having an inlet therein;
an outlet member (104) comprising an outlet therein,
a conduit (132) which has a distal end (134) associated with the inlet member (102) and a proximal end (136) associated with the outlet member (104),
a support (116) associated with the conduit (132) that is adjustable between an expanded state and a contracted state whereby in the contracted state the inlet member (102) is positioned proximate the outlet member (104), while in the expanded state the support (116) supports the conduit (132) and combined with the inlet member (102) being spaced from the outlet member (104) that defines a chamber (130),
so that in use a metered dose of medicament can be dispensed from the MDI dispenser through the inlet in the inlet member (102) to mix with air in the chamber (130) and be inhaled by a patient through the outlet in the outlet member (104);
**characterised in that**: the inhaler (100) further comprises a lockout mechanism (118) for inhibiting operation of the MDI dispenser when the support (116) and conduit (132) are in the contracted state and not inhibiting operation of the MDI dispenser when the support (116) and conduit (132) are in the expanded state; and the conduit (132) is pliable.

2. The inhaler (100) of claim 1, wherein the MDI dispenser comprises an MDI canister (106) containing the medicament and a needle valve (126) for dispensing the medicament there through, wherein the valve opens upon depression of the MDI canister (106) by engagement with a saddle (128) associated with the inlet member (102).

3. The inhaler (100) of claim 1, wherein the MDI dispenser comprises an MDI canister (106) containing the medicament and a needle valve (126) for dispensing the medicament there through, and further comprises an actuator comprising a saddle (128) and a mouth piece (122), wherein the valve of the MDI canister (106) opens upon depression of the MDI canister (106) by engagement with the actuator saddle (128).

4. The inhaler (100) of any one of claims 2 or 3, wherein the lockout mechanism (118) inhibits depression of the MDI canister (106) when the support (116) and conduit (132) are in the contracted state and does not inhibit depression of the MDI canister (106) when the support (116) and conduit (132) are in the expanded state.

5. The inhaler (100) of any one of claims 2 to 4, wherein the lockout mechanism (118) comprises an elongate member (120) extending from the outlet member (104) to engage and act as a wedge between the MDI canister (106) and the saddle (128) of the inlet member (102), or the MDI canister (106) and the saddle of the actuator (196), to inhibit depression of MDI canister (106) when the support (116) and conduit (132) are in the contacted state, and not engage the MDI canister (106) and saddle (128) to not inhibit depression of the MDI canister (106) when the support (116) and conduit (132) are in the expanded state.

6. The inhaler (100) of any one of claims 2 to 5, wherein the inlet member (102) and outlet member (104) are adapted to realisably engage each other when the support (116) is in the contracted state, wherein the inlet member (102) realisably engages the outlet member (104) by way of a bayonet fitting (158).

7. The inhaler (100) of any one of claims 1 to 6, wherein the inlet member (102) is adapted to receive the MDI dispenser by way of a tube shaped appendage (182) that is complementary in shape to the MDI dispenser such that an at least partial seal is formed in use.

8. The inhaler (100) of any one of claims 2 to 7, wherein the inlet member (102) further comprises a mount (148) wherein, the saddle (128) associated with the inlet member (102) to engage the MDI dispenser and enable actuation of the MDI dispenser upon depression is positioned on the mount (148).

9. The inhaler (100) of any one of claims 3 to 8, wherein the inlet member (102) is adapted to receive the mouth piece of the MDI dispenser by having a complementary shaped recess, the recess comprising an aperture and having a shape complementary the mouthpiece of the MDI dispenser such that an at least partial seal is formed in use.

10. The inhaler (100) of any one of claims 2 to 9, wherein the saddle (128) of the inlet member (102), or saddle of the actuator (196) of the MDI dispenser, engages the MDI dispenser at an angle relative to the conduit (132), or wherein the recess engages the MDI dispenser at an angle relative to the chamber (130) such that release of the medicament substantially is parallel an axis of the chamber (130).

11. The inhaler (100) of any one of claims 1 to 10, wherein the outlet member (104) is lenticular having a concave surface facing interior to the chamber (130), the concave surface having a second association portion for associating with the proximal end of the conduit (132) and support (116).

12. The inhaler (100) of any one of claims 1 to 11, wherein the support (116) is a helical spring (142), and wherein the spring (142) has a tension such that the conduit (132) in the expanded state is about linear along an axis parallel to the chamber (130), such that the weight of the outlet member (104) and inlet member (102) do not substantially deform the conduit (132) in the expanded state.

13. A system for respiratory delivery of a medicament, the system comprising the inhaler of claims 1 to 12 and an MDI dispenser, wherein the MDI dispenser contains a medicament composition comprising a pharmaceutically active agent.

14. The inhaler of any one of claims 1-12 for use in treating chronic obstructive pulmonary disease (COPD), asthma and related disorders or transient bronchospasm.

15. The system of claim 13 for use in treating chronic obstructive pulmonary disease (COPD), asthma and related disorders or transient bronchospasm.

## Patentansprüche

1. Zusammenklappbarer Inhalator (100) zum Gebrauch mit einem Dosierinhalator-, (MDI)-, Spender, der betreibbar ist, um eine abgemessene Dosis eines Medikaments daraus abzugeben, wobei der Inhalator (100) Folgendes umfasst:
ein Einlasselement (102), das zur Aufnahme eines MDI-Spenders ausgelegt ist und einen Einlass darin aufweist;
ein Auslasselement (104), das einen Auslass darin umfasst,
eine Leitung (132), die ein distales Ende (134) aufweist, das dem Einlasselement (102) zugeordnet ist, und ein proximales Ende (136), das dem Auslasselement (104) zugeordnet ist,
einen der Leitung (132) zugeordneten Träger (116), der zwischen einem expandierten Zustand und einem kontrahierten Zustand einstellbar ist, wobei in dem kontrahierten Zustand das Einlasselement (102) nahe dem Auslasselement (104) positioniert ist, während in dem expandierten Zustand der Träger (116) die Leitung (132) trägt und zusammen mit dem Einlasselement (102), das von dem Auslasselement (104) beabstandet ist, eine Kammer (130) definiert,
so dass im Gebrauch eine abgemessene Medikamentendosis aus dem MDI-Spender durch den Einlass in das Einlasselement (102) abgegeben werden kann, um sich mit der Luft in der Kammer (130) zu mischen und von einem Patienten durch den Auslass in dem Auslasselement (104) inhaliert zu werden;
**dadurch gekennzeichnet, dass**: der Inhalator (100) weiter einen Sperrmechanismus (118) umfasst, um den Betrieb des MDI-Spenders zu hemmen, wenn sich der Träger (116) und die Leitung (132) in dem kontrahierten Zustand befinden, und den Betrieb des MDI-Spenders nicht zu hemmen, wenn sich der Träger (116) und die Leitung (132) in dem expandierten Zustand befinden; und die Leitung (132) biegsam ist.

2. Inhalator (100) nach Anspruch 1, wobei der MDI-Spender einen MDI-Behälter (106), der das Medikament enthält, und ein Nadelventil (126) zur Abgabe des Medikaments durch diesen hindurch umfasst, wobei sich das Ventil beim Niederdrücken des MDI-Behälters (106) durch Eingriff mit einem Sattel (128) öffnet, der dem Einlasselement (102) zugeordnet ist.

3. Inhalator (100) nach Anspruch 1, wobei der MDI-Spender einen MDI-Behälter (106), der das Medikament enthält, und ein Nadelventil (126) zur Abgabe des Medikaments durch diesen hindurch umfasst, und weiter einen Aktuator umfasst, der einen Sattel (128) und ein Mundstück (122) umfasst, wobei sich das Ventil des MDI-Behälters (106) beim Niederdrücken des MDI-Behälters (106) durch Eingriff mit dem Aktuatorsattel (128) öffnet.

4. Inhalator (100) nach einem der Ansprüche 2 oder 3, wobei der Sperrmechanismus (118) das Niederdrücken des MDI-Behälters (106) hemmt, wenn sich der Träger (116) und die Leitung (132) im kontrahierten Zustand befinden, und das Niederdrücken des MDI-Behälters (106) nicht hemmt, wenn sich der Träger (116) und die Leitung (132) im expandierten Zustand befinden.

5. Inhalator (100) nach einem der Ansprüche 2 bis 4, wobei der Sperrmechanismus (118) ein längliches Element (120) umfasst, das sich von dem Auslasselement (104) erstreckt, um den MDI-Behälter (106) und den Sattel (128) des Einlasselements (102) oder den MDI-Behälter (106) und den Sattel des Aktuators (196) in Eingriff zu bringen und dazwischen als Keil zu wirken, um das Niederdrücken des MDI-Behälters (106) zu hemmen, wenn sich der Träger (116) und die Leitung (132) in dem kontrahierten Zustand befinden, und den MDI-Behälter (106) und den Sattel (128) nicht in Eingriff zu bringen, um das Niederdrücken des MDI-Behälters (106) nicht zu hemmen, wenn sich der Träger (116) und die Leitung (132) in dem expandierten Zustand befinden.

6. Inhalator (100) nach einem der Ansprüche 2 bis 5, wobei das Einlasselement (102) und das Auslasselement (104) so ausgelegt sind, dass sie realisierbar ineinander eingreifen, wenn sich der Träger (116) im kontrahierten Zustand befindet, wobei das Einlasselement (102) über einen Bayonettverschluss (158) realisierbar in das Auslasselement (104) eingreift.

7. Inhalator (100) nach einem der Ansprüche 1 bis 6, wobei das Einlasselement (102) so ausgelegt ist, dass es den MDI-Spender mittels eines röhrenförmigen Ansatzes (182) aufnimmt, der in der Gestalt komplementär zu dem MDI-Spender ist, so dass im Gebrauch eine zumindest teilweise Abdichtung gebildet wird.

8. Inhalator (100) nach einem der Ansprüche 2 bis 7, wobei das Einlasselement (102) weiter eine Halterung (148) umfasst, wobei der dem Einlasselement (102) zugeordnete Sattel (128) auf der Halterung (148) positioniert ist, um in den MDI-Spender einzugreifen und die Betätigung des MDI-Spenders beim Niederdrücken zu ermöglichen.

9. Inhalator (100) nach einem der Ansprüche 3 bis 8, wobei das Einlasselement (102) so ausgelegt ist, dass es das Mundstück des MDI-Spenders aufnimmt, indem es eine komplementär geformte Aussparung aufweist, wobei die Aussparung eine Öffnung umfasst und eine zum Mundstück des MDI-Spenders komplementäre Gestalt aufweist, so dass im Gebrauch eine zumindest teilweise Abdichtung gebildet wird.

10. Inhalator (100) nach einem der Ansprüche 2 bis 9, wobei der Sattel (128) des Einlasselements (102) oder der Sattel des Aktuators (196) des MDI-Spenders in einem Winkel relativ zur Leitung (132) in den MDI-Spender eingreift, oder wobei die Aussparung in einem Winkel relativ zur Kammer (130) in den MDI-Spender eingreift, so dass die Freisetzung des Medikaments im Wesentlichen parallel zu einer Achse der Kammer (130) erfolgt.

11. Inhalator (100) nach einem der Ansprüche 1 bis 10, wobei das Auslasselement (104) linsenförmig ist und eine konkave Oberfläche aufweist, die dem Inneren der Kammer (130) zugewandt ist, wobei die konkave Oberfläche einen zweiten Zuordnungsabschnitt aufweist, der dem proximalen Ende der Leitung (132) und dem Träger (116) zugeordnet ist.

12. Inhalator (100) nach einem der Ansprüche 1 bis 11, wobei der Träger (116) eine Schraubenfeder (142) ist, und wobei die Feder (142) eine solche Spannung aufweist, dass die Leitung (132) im expandierten Zustand entlang einer Achse parallel zur Kammer (130) ungefähr linear ist, so dass das Gewicht des Auslasselements (104) und des Einlasselements (102) die Leitung (132) im expandierten Zustand nicht im Wesentlichen verformt.

13. System zur respiratorischen Abgabe eines Medikaments, wobei das System den Inhalator nach den Ansprüchen 1 bis 12 und einen MDI-Spender umfasst, wobei der MDI-Spender eine Medikamentenzusammensetzung enthält, die einen pharmazeutischen Wirkstoff umfasst.

14. Inhalator nach einem der Ansprüche 1-12 zum Gebrauch bei der Behandlung von chronisch obstruktiver pulmonaler Erkrankung (COPD), Asthma und verwandten Erkrankungen oder vorübergehendem Bronchospasmus.

15. System nach Anspruch 13 zum Gebrauch bei der Behandlung von chronisch obstruktiver pulmonaler Erkrankung (COPD), Asthma und verwandten Erkrankungen oder vorübergehendem Bronchospasmus.

## Revendications

1. Inhalateur pliable (100) destiné à être utilisé avec un distributeur d'inhalateur doseur (MDI) qui permet de distribuer une dose mesurée de médicament à partir de celui-ci, l'inhalateur (100) comprenant :
un élément d'entrée (102) qui est adapté pour recevoir un distributeur d'inhalateur MDI et présentant une entrée ;
un élément de sortie (104) comprenant une sortie,
un conduit (132) qui présente une extrémité distale (134) associée à l'élément d'entrée (102) et une extrémité proximale (136) associée à l'élément de sortie (104),
un support (116) associé au conduit (132) qui est réglable entre un état déployé et un état contracté, selon lequel, dans l'état contracté, l'élément d'entrée (102) est positionné à proximité de l'élément de sortie (104), tandis que, dans l'état déployé, le support (116) supporte le conduit (132) et est combiné à l'élément d'entrée (102) qui est espacé de l'élément de sortie (104) qui définit une chambre (130),
de telle sorte que lors de l'utilisation, une dose mesurée de médicament peut être distribuée à partir du distributeur d'inhalateur MDI par l'entrée dans l'élément d'entrée (102) pour se mélanger à de l'air dans la chambre (130) et être inhalée par un patient par la sortie dans l'élément de sortie (104) ;
**caractérisé en ce que** : l'inhalateur (100) comprend en outre un mécanisme de verrouillage (118) pour entraver le fonctionnement du distributeur d'inhalateur MDI lorsque le support (116) et le conduit (132) sont dans l'état contracté et pour ne pas entraver le fonctionnement du distributeur d'inhalateur MDI lorsque le support (116) et le conduit (132) sont dans l'état déployé ; et le conduit (132) est pliable.

2. Inhalateur (100) selon la revendication 1, dans lequel le distributeur d'inhalateur MDI comprend une cartouche d'inhalateur MDI (106) contenant le médicament et une valve à aiguille (126) pour distribuer le médicament à travers celle-ci, dans lequel la valve s'ouvre lors de l'enfoncement de la cartouche d'inhalateur MDI (106) par mise en prise avec une selle (128) associée à l'élément d'entrée (102).

3. Inhalateur (100) selon la revendication 1, dans lequel le distributeur d'inhalateur MDI comprend une cartouche d'inhalateur MDI (106) contenant le médicament et une valve à aiguille (126) pour distribuer le médicament à travers celle-ci, et comprend en outre un actionneur comprenant une selle (128) et un embout buccal (122), dans lequel la valve de la cartouche d'inhalateur MDI (106) s'ouvre lors de l'enfoncement de la cartouche d'inhalateur MDI (106) par mise en prise avec la selle d'actionneur (128).

4. Inhalateur (100) selon l'une quelconque des revendications 2 ou 3, dans lequel le mécanisme de verrouillage (118) entrave l'enfoncement de la cartouche d'inhalateur MDI (106) lorsque le support (116) et le conduit (132) sont dans l'état contracté et n'entrave pas l'enfoncement de la cartouche d'inhalateur MDI (106) lorsque le support (116) et le conduit (132) sont dans l'état déployé.

5. Inhalateur (100) selon l'une quelconque des revendications 2 à 4, dans lequel le mécanisme de verrouillage (118) comprend un élément allongé (120) s'étendant depuis l'élément de sortie (104) pour venir en prise et agir comme un coin entre la cartouche d'inhalateur MDI (106) et la selle (128) de l'élément d'entrée (102), ou la cartouche d'inhalateur MDI (106) et la selle de l'actionneur (196), pour entraver l'enfoncement de la cartouche d'inhalateur MDI (106) lorsque le support (116) et le conduit (132) ) sont dans l'état contracté et ne viennent pas en prise avec la cartouche d'inhalateur MDI (106) et la selle (128) pour ne pas entraver l'enfoncement de la cartouche d'inhalateur MDI (106) lorsque le support (116) et le conduit (132) sont dans l'état déployé.

6. Inhalateur (100) selon l'une quelconque des revendications 2 à 5, dans lequel l'élément d'entrée (102) et l'élément de sortie (104) sont adaptés pour s'engager de manière réalisable l'un dans l'autre lorsque le support (116) est dans l'état contracté, dans lequel l'élément d'entrée (102) vient en prise de manière réalisable avec l'élément de sortie (104) au moyen d'un raccord à baïonnette (158).

7. Inhalateur (100) selon l'une quelconque des revendications 1 à 6, dans lequel l'élément d'entrée (102) est adapté pour recevoir le distributeur d'inhalateur MDI au moyen d'un appendice en forme de tube (182) qui est de forme complémentaire au distributeur d'inhalateur MDI de telle sorte qu'une étanchéité au moins partielle soit formée lors de l'utilisation.

8. Inhalateur (100) selon l'une quelconque des revendications 2 à 7, dans lequel l'élément d'entrée (102) comprend en outre un support (148) dans lequel la selle (128) associée à l'élément d'entrée (102) pour venir en prise avec le distributeur d'inhalateur MDI et permettre l'actionnement du distributeur d'inhalateur MDI lors de l'enfoncement, est positionnée sur le support (148).

9. Inhalateur (100) selon l'une quelconque des revendications 3 à 8, dans lequel l'élément d'entrée (102) est adapté pour recevoir l'embout buccal du distributeur d'inhalateur MDI en présentant un évidement de forme complémentaire, l'évidement comprenant un orifice et présentant une forme complémentaire à celle de l'embout buccal du distributeur d'inhalateur MDI de telle sorte qu'une étanchéité au moins partielle soit formée lors de l'utilisation.

10. Inhalateur (100) selon l'une quelconque des revendications 2 à 9, dans lequel la selle (128) de l'élément d'entrée (102), ou la selle de l'actionneur (196) du distributeur d'inhalateur MDI, vient en prise avec le distributeur d'inhalateur MDI selon un angle par rapport au conduit (132), ou dans lequel l'évidement vient en prise avec le distributeur d'inhalateur MDI selon un angle par rapport à la chambre (130) de telle sorte que la libération du médicament soit sensiblement parallèle à un axe de la chambre (130).

11. Inhalateur (100) selon l'une quelconque des revendications 1 à 10, dans lequel l'élément de sortie (104) est lenticulaire présentant une surface concave tournée vers l'intérieur de la chambre (130), la surface concave présentant une seconde partie d'association pour être associée à l'extrémité proximale du conduit (132) et du support (116).

12. Inhalateur (100) selon l'une quelconque des revendications 1 à 11, dans lequel le support (116) est un ressort hélicoïdal (142) et dans lequel le ressort (142) présente une tension telle que le conduit (132) à l'état déployé, soit à peu près linéaire, le long d'un axe parallèle à la chambre (130), de telle sorte que le poids de l'élément de sortie (104) et de l'élément d'entrée (102) ne déforme pas sensiblement le conduit (132) à l'état déployé.

13. Système pour l'administration respiratoire d'un médicament, le système comprenant l'inhalateur selon les revendications 1 à 12 et un distributeur d'inhalateur MDI, dans lequel le distributeur d'inhalateur MDI contient une composition médicamenteuse comprenant un agent pharmaceutiquement actif.

14. Inhalateur selon l'une quelconque des revendications 1-12, destiné à être utilisé dans le traitement de la maladie pulmonaire chronique obstructive (MPOC), de l'asthme et de troubles associés ou du bronchospasme transitoire.

15. Système selon la revendication 13, destiné à être utilisé dans le traitement de la maladie pulmonaire chronique obstructive (MPOC), de l'asthme et de troubles associés ou du bronchospasme transitoire.
